# EUROPÄISCHE PATENTANMELDUNG

(11) **EP 1 787 989 A1**
(43) Veröffentlichungstag der Anmeldung: **23.05.2007**
(21) Anmeldenummer: 05110842.1
(22) Anmeldetag: 17.11.2005
(51) Int. Cl.: C07D 401/14, C07D 401/12, C07D 251/70

(54) **Triazinverbindungen mit Aminogruppen- und Carboxygruppen-haltigen Substituenten**

(71) Anmelder: Degussa GmbH, 40474 Düsseldorf (DE)
(72) Erfinder: Kirchhoff, Jochen, Dr., 59348, Lüdinghausen (DE); Werle, Peter, Dr., 63571 Gelnhausen (DE); Kreilkamp, Günter, 45768, Marl (DE); Kruppa, Edeltraud, 45699, Herten (DE)

(57) **Zusammenfassung**

Die Erfindung betrifft Triazinverbindungen der Formel 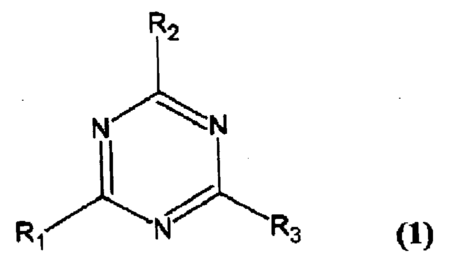 mit den Bedeutungen für R¹, R² und R³ gemäß Anspruch 1, die sowohl Amino- als auch Carboxygruppenhaltige Substituenten aufweisen, sowie ein Verfahren zu deren Herstellung.

## Beschreibung

Die Erfmdung betrifft Triazinverbindungen, die sowohl Amino- als auch Carboxygruppen-haltige Substituenten aufweisen, sowie ein Verfahren zu deren Herstellung.

Die EP 0 466 647 beschreibt ein Verfahren zur photochemischen und thermischen Stabilisierung von Polyamid-Fasermaterialien sowie wasserlösliche Triazinderivate gemäß der Formel I.

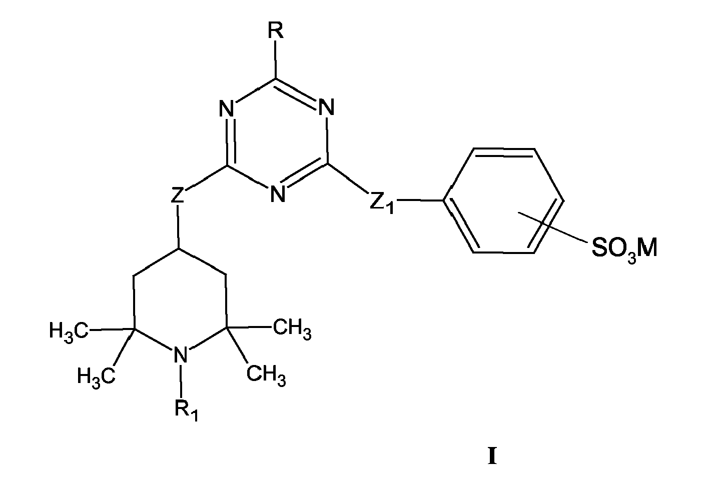

Ebenfalls ein Verfahren zur Verbesserung thermischer und/oder photochemischer Stabilität von ungefärbten und gefärbten Polyamidfasern beschreibt die DE 195 31 995. Hierbei wird die Stabilisierung durch Behandlung mit einem Mittel aus wässrigen Bad, das eine Verbindung der Formel II: 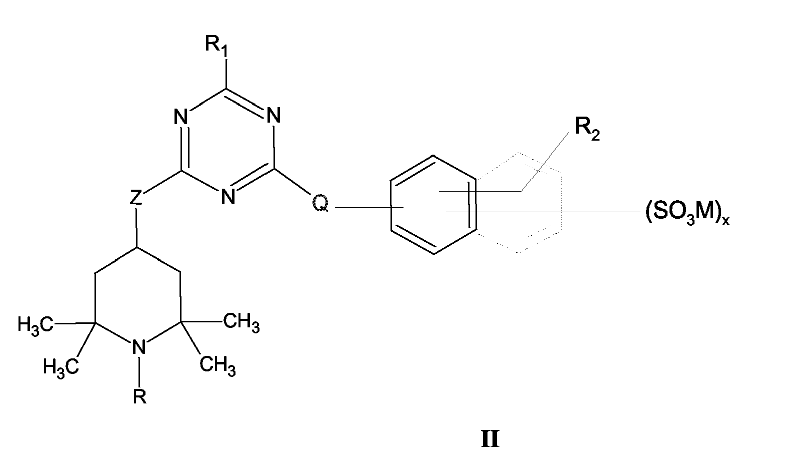 und einen UV-Absorber enthält, erzielt.

Auch die EP 0 702 011 beschreibt ein Verfahren zur photochemischen und thermischen Stabilisierung von Polyamid-Fasermaterialien und -färbungen. Hierfür werden wasserlösliche Piperidin Triazinverbindungen der Formel III 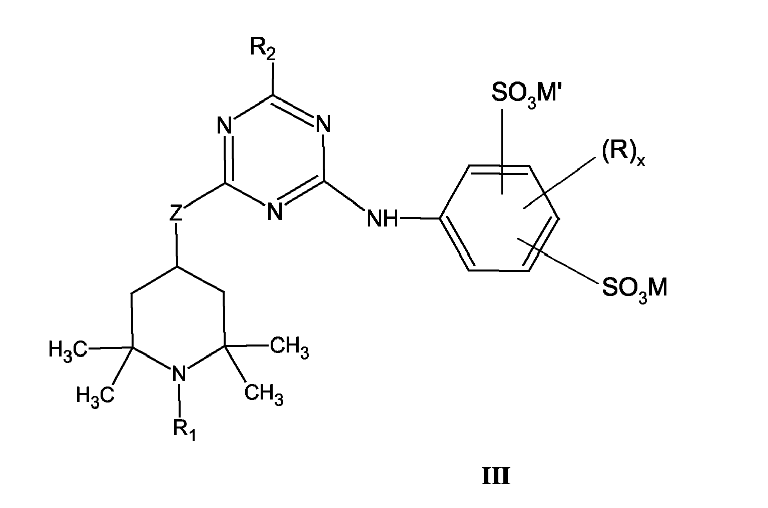 eingesetzt.

Ähnliche Verbindungen zur photochemischen und thermischen Stabilisierung von Polyamidfasermaterialien beschreibt auch die EP 0 546 993. Verbindung gemäß der Formel IV 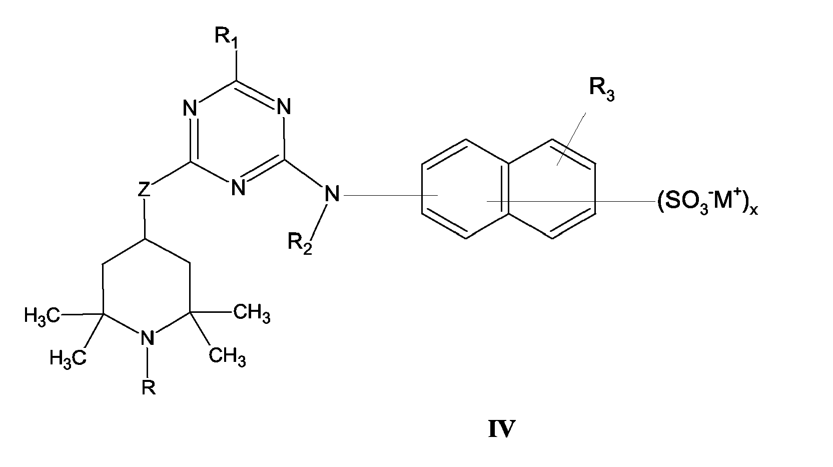 werden hierfür eingesetzt.

US 4,883,860 beschreibt polymere Zusammensetzungen, die eine wirksame Menge einer Verbindung auf Triazinbasis, die eine 2,2,6,6-Tetraalkylpiperidingruppe aufweist, wie beispielsweise gemäß der Formel V.

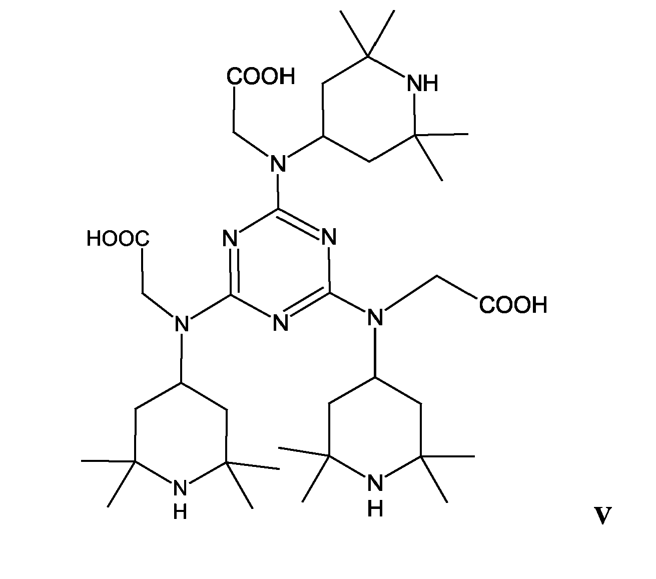

Es war die Aufgabe der vorliegenden Erfindung Verbindungen zur Verfügung zu stellen, die für den Einsatz als permanent wirkende Stabilisatoren für Polyester und Polyamide geeignet sind. Insbesondere sollten diese Verbindungen wirtschaftlich, aus kommerziell erhältlichen Rohstoffen durch wenige Reaktionsschritte herstellbar sein.

Überraschenderweise wurde gefunden, dass Triazinverbindungen, die sowohl Amino- als auch Carboxygruppen-haltige Substituenten aufweisen, als reaktive Stabilisatoren für Polyester und Polyamide geeignet sind. Unter reaktiven Stabilisatoren wird im Sinne dieser Erfmdung verstanden, dass die erfmdungsgemäßen Stabilisatoren durch ihre Amino- und Carboxygruppen-haltige Substituenten in der Lage sind, in das Polymer eingebunden zu werden und damit ein Baustein in der Polymerkette sind. Dies hat den Vorteil gegenüber Stabilisatoren gemäß dem Stand der Technik, dass die erfindungsgemäßen Stabilisatoren bereits bei der Polymerisation zugefügt und in die Polymerkette eingebunden werden können. Somit kann der zusätzliche Reaktionsschritt für die Beimischung des Stabilisators zu dem Polymer entfallen. Weiterhin haben diese erfindungsgemäßen Stabilisatoren den Vorteil, dass diese nicht aus dem Polymer herausgelöst werden können und somit ein permanent wirksamer Stabilisator zur Verfügung steht. Die Lösung der Aufgabe war umso überraschender, zumal sich zeigte, dass diese Verbindung gemäß eines wirtschaftlichen Verfahrens hergestellt werden können.

Gegenstand der Erfmdung sind Triazinverbindungen der Formel (1) 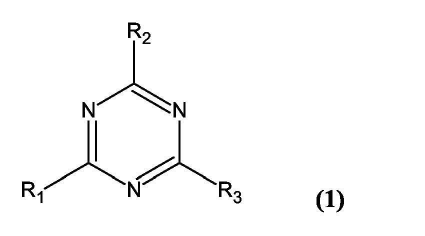 wobei
R₁ = -A-B mit A = -O- oder -NR₄-,
B = Aminogruppe-haltiger Substituent und
R₄ = Wasserstoff oder Alkylgruppe
R_{2 =} 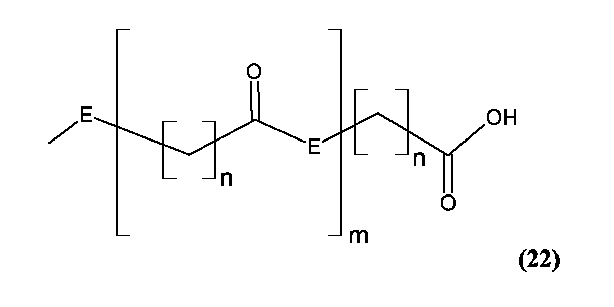 mit E = -O- oder -NR₅-,
n = 3 bis 15,
m = 0 bis 10 und
R₅ = Wasserstoff oder Alkylgruppe
R₃ = R₁, R₂, -OR₆ oder -NR₇R₈
mit R₆, R₇ und R₈ = Wasserstoff, Alkyl- oder Arylgruppe, die jeweils substituiert oder unsubstituiert ist.
ist.

Ebenso ist Gegenstand dieser Erfindung ein Verfahren zur Herstellung von Triazinverbindungen gemäß Formel (1), das sich dadurch auszeichnet, dass Cyanurchlorid mit von 0,5 bis 5 Moläquivalenten eines Amins der Formel (2)

H-A-B (2)

in Gegenwart einer Base und von 0,5 bis 5 Moläquivalenten einer Verbindung gemäß der Formel (3) 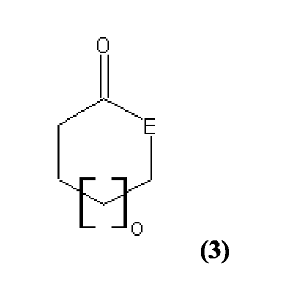 oder einer Verbindung gemäß der Formel (4) 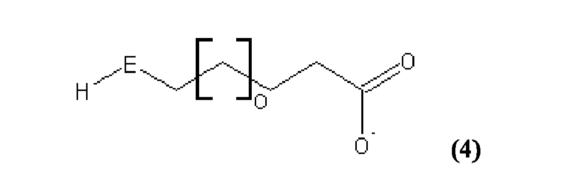 mit o von 0 bis 12,
E = -O- oder -NR₅- und
R₅ = Wasserstoff oder Alkylgruppe,
umgesetzt wird, wobei die Reihenfolge der beiden Reaktionsschritte beliebig ist.

Ferner ist Gegenstand dieser Erfindung eine Zusammensetzung, die dadurch gekennzeichnet ist, dass die Zusammensetzung zumindest zwei verschiedene Triazinverbindungen gemäß der Formel (1) aufweist.

Des weiteren ist Gegenstand dieser Erfindung eine Lösung, welche dadurch gekennzeichnet ist, dass die Lösung zumindest eine Triazinverbindung gemäß der Formel (1) aufweist.

Die erfindungsgemäßen Triazinverbindungen weisen eine Struktur gemäß der Formel (1) 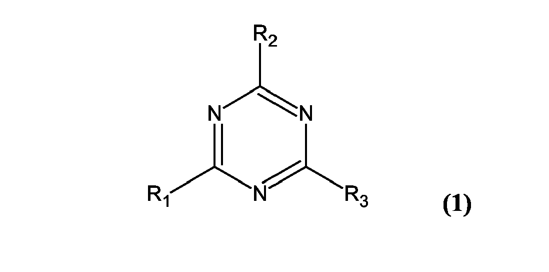 wobei
R₁ = -A-B mit A = -O- oder -NR₄-,
B = Aminogruppe-haltiger Substituent und
R₄ = Wasserstoff oder Alkylgruppe
R₂ = 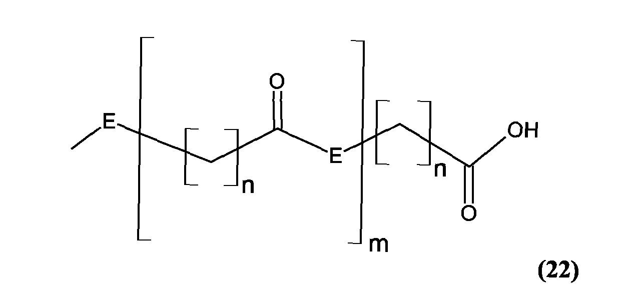 mit E = -O- oder -NR₅-,
n = 3 bis 15,
m = 0 bis 10 und
R₅ = Wasserstoff oder Alkylgruppe
R₃ = R₁, R₂, -OR₆ oder -NR₇R₈
mit R₆, R₇ und R₈ = Wasserstoff, Alkyl- oder Arylgruppe, die jeweils substituiert oder unsubstituiert ist,
ist, auf.

Das Strulcturfragment A in dem Substituenten vom Typ R₁ der erfindungsgemäßen Triazinverbindungen kann sowohl -O- als auch -NR₄- sein, wobei das Strukturfragment A in den Substituenten vom Typ R₁ und R₃ gleich oder verschieden sein kann. Bevorzugt ist das Strukturfragment A -NR₄-. Der Substituent vom Typ R₄ kann sowohl Wasserstoff als auch eine Alkylgruppe sein. Vorzugsweise ist der Substituent vom Typ R₄ Wasserstoff oder eine Alkylgruppe mit einer Kohlenstoffanzahl von 1 bis 10, bevorzugt von 2 bis 5. Diese Alkylgruppe des Strukturfragments A kann verzweigt oder unverzweigt sein, bevorzugt ist diese jedoch unverzweigt. Ferner ist diese Alkylgruppe vorzugsweise unsubstituiert. Besonders bevorzugt ist der Substituent vom Typ R₄ jedoch Wasserstoff.

Der Substituent vom Typ B ist insbesondere ein Aminogruppe-haltiger Substituent, wobei die Aminogruppe sich an einem aliphatischen Grundgerüst befinden kann oder die Aminogruppe ein aliphatisches cyclisches Amin sein kann. Bevorzugt weist der Substituent vom Typ B ein aliphatisches cyclisches Amin auf.

Vorzugsweise weisen die erfindungsgemäßen Triazinverbindungen einen Substituenten vom Typ B gemäß der Formel (5) 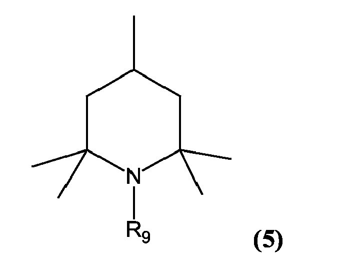 wobei R₉ = Wasserstoff, Alkyl- oder eine Alkoxygruppe der Formel -O-R₂₀,
R₂₀ = verzweigte oder unverzweigte Alkyl- oder Cycloalkylgruppe mit jeweils einer Kohlenstoffanzahl von 4 bis 16, ist,
oder gemäß der Formel (6)

-(CH₂)ₚ-NR₁₀R₁₁ (6)

wobei p von 1 bis 15, bevorzugt von 2 bis 8 und besonders bevorzugt von 3 bis 6, ist,
in dem Substituenten vom Typ R₁ auf.

Die Substituenten vom Typ R₁₀ und R₁₁ können gleich oder verschieden sein und sind vorzugsweise Wasserstoff, eine Alkyl-, eine Cycloalkyl- oder eine Heterocycloalkylgruppe, insbesondere mit jeweils einer Kohlenstoffanzahl von 1 bis 20, bevorzugt von 2 bis 10 bzw. einer Anzahl an Kohlenstoffatomen und Heteroatomen von 1 bis 20, bevorzugt von 2 bis 10. Diese Alkylgruppe der Substituenten vom Typ R₁₀ und R₁₁ sind vorzugsweise verzweigt oder unverzweigt, bevorzugt sind diese jedoch unverzweigt. Ferner sind sie vorzugsweise unsubstituiert oder mit einer Aminogruppe substituiert, bevorzugt ist sie jedoch unsubstituiert. Die Cycloalkylgruppe der Substituenten vom Typ R₁₀ und R₁₁ ist vorzugsweise unsubstituiert oder substituiert, insbesondere ist diese Cycloalkylgruppe unsubstituiert. Die Heterocycloalkylgruppe der Substituenten vom Typ R₁₀ und R₁₁ ist vorzugsweise unsubstituiert oder substituiert, bevorzugt ist diese Heterocycloalkylgruppe mit ein oder mehreren Methylgruppen substituiert, bevorzugt ist sie eine Heterocycloalkylgruppe, die als Heteroatom ein oder mehrere Stickstoffatome aufweist, bevorzugt ist sie eine Heterocycloalkylgruppe gemäß der Formel (5).

Der Substituent vom Typ R₉ ist vorzugsweise Wasserstoff oder eine Alkylgruppe mit einer Kohlenstoffanzahl von 1 bis 16, bevorzugt von 1 bis 8 oder eine Alkoxygruppe mit einer verzweigten oder unverzweigten Alkylgruppe oder einer Cycloalkygruppe, besonders bevorzugt ist der Substituent vom Typ R₉ Wasserstoff.

Bevorzugt weisen die erfindungsgemäßen Triazinverbindungen einen Substituenten vom Typ B gemäß der Formel (6) auf. Besonders bevorzugt weisen die erfindungsgemäßen Triazinverbindung einen Substituenten vom Typ B gemäß den Formeln (6a) oder (6b) auf:

-(CH₂)₃-NEt₂ **(6a)**

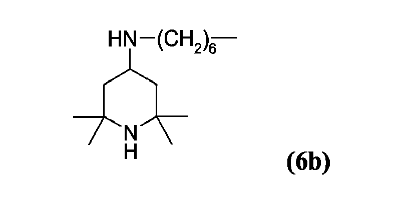

In einer besonders bevorzugten Ausführungsform der erfmdungsgemäßen Triazinverbindungen weisen diese einen Substituenten vom Typ B gemäß der Formel (5) auf.

Ganz besonders bevorzugt sind Triazinverbindungen mit einem Substituenten vom Typ R₁ gemäß Formel (7):

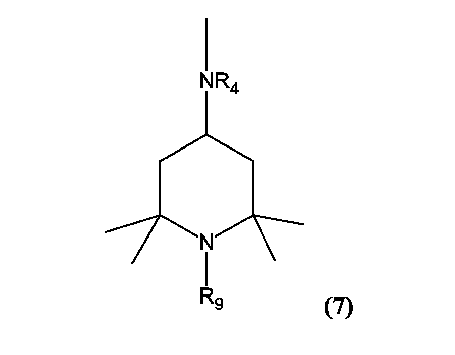

Die Substituenten vom Typ R₂ in den erfindungsgemäßen Triazinverbindungen weisen vorzugsweise ein Strukturfragment E mit -O- oder -NR₅- auf, wobei der Substituent vom Typ R₅ vorzugsweise Wasserstoff oder eine Alkylgruppe mit einer Kohlenstoffanzahl von 1 bis 16, bevorzugt von 1 bis 4 ist, bevorzugt ist der Substituent vom Typ R₅ Wasserstoff. Die Alkylgruppe des Substituenten vom Typ R₅ ist verzweigt oder unverzweigt, bevorzugt ist diese jedoch unverzweigt. Ferner ist diese Alkylgruppe des Substituenten vom Typ R₅ vorzugsweise unsubstituiert. In dem Substituenten vom Typ R₂ ist n vorzugsweise von 3 bis 15, bevorzugt von 5 bis 11 und besonders bevorzugt 5 und m ist vorzugsweise von 0 bis 10, bevorzugt von 0 bis 4 und besonders bevorzugt ist m gleich 0.

Die erfindungsgemäßen Triazinverbindungen weisen vorzugsweise als Substituenten vom Typ R₃ einen Substituenten vom Typ R₁ auf In einer besonderen Ausführungsform weisen die erfindungsgemäßen Triazinverbindungen als Substituenten vom Typ R₃ einen Substituenten vom Typ R₂ auf. Die beiden Substituenten vom Typ R₁ bzw. R₂ können identisch oder verschieden sein, bevorzugt sind die Substituenten vom selben Typ identisch.

In einer bevorzugten Ausführungsform weisen die erfindungsgemäßen Triazinverbindungen einen Substituenten vom Typ R₁ gemäß den folgenden Strukturen (7a), (7b) oder (7c) auf:

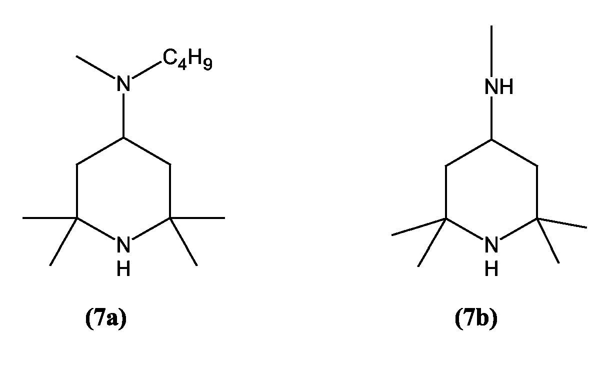

-NH-(CH₂)₃-NEt₂ **(7c)**

Die Substituenten vom Typ R₂ weisen in dieser bevorzugten Ausführungsform insbesondere Strukturen gemäß den Formeln (8a) oder (8b) auf: 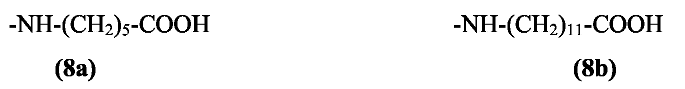 In dieser bevorzugten Ausführungsform ist der Substituent vom Typ R₃ vorzugsweise ein Substituent vom Typ R₁ und besonders bevorzugt sind diese beiden Substituenten identisch. Der Substituent vom Typ R₃ kann in dieser bevorzugten Ausführungsform auch ein Substituent vom Typ R₂ sein, auch hier sind diese beiden Substituenten vorzugsweise identisch.

In einer weiteren Ausführungsform der erfindungsgemäßen Triazinverbindungen weisen diese einen Substituten vom Typ R₃ gemäß den folgenden Formeln (14) und (15) auf: 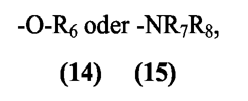 wobei R₆, R₇ und R₈ Wasserstoff, Alkyl- oder Arylgruppe ist, wobei die Alkyl- oder Arylgruppe unsubstituiert oder mit einem oder mehreren Substituenten der Formel R₆, R₇, R₈, -SO₃H oder -SO₃M, wobei M ein Alkalimetallkation, bevorzugt ein Lithium-, Natrium- oder Kaliumkation ist, substituiert ist. Die Alkyl- und/oder Arylgruppen der Substituenten vom Typ R₆, R₇ oder R₈ weisen bevorzugt von 1 bis 4, besonders bevorzugt von 1 bis 2 und ganz besonders bevorzugt einen Substituenten der Formel -SO₃H oder -SO₃M auf. Vorzugsweise sind die Substituenten vom Typ R₆, R₇ und R₈ Wasserstoff oder eine Alkylgruppe mit einer Kohlenstoffanzahl von 1 bis 18, bevorzugt von 2 bis 16. Diese Alkylgruppe des Substituenten vom Typ R₆, R₇ und R₈ kann verzweigt oder unverzweigt sein, bevorzugt ist diese jedoch unverzweigt. Die Arylgruppe der Substituenten vom Typ R₆, R₇ und R₈ ist vorzugsweise eine Phenylgruppe oder eine mit -SO₃H oder -SO₃M einfach substituierte Phenylgruppe, bevorzugt in para-Stellung. Die Substituenten vom Typ R₆, R₇ und R₈ können alle identisch, alle verschieden oder auch paarweise identisch sein. Bevorzugt sind die Substituenten vom Typ R₆, R₇ und R₈ Wasserstoff, Phenyl- oder eine mit -SO₃H oder -SO₃M in para-Stellung einfach substituierte Phenylgruppe.

Das erfindungsgemäße Verfahren zur Herstellung von Triazinverbindungen gemäß Formel (1), 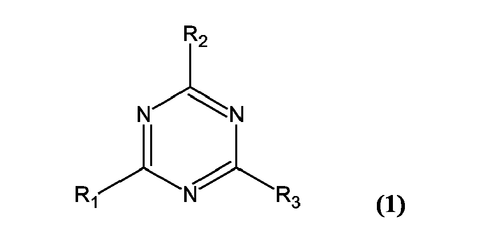 wobei
R₁ = -A-B mit A = -O- oder -NR₄-,
B = Aminogruppe-haltiger Substituent und
R₄ = Wasserstoff oder Alkylgruppe
R₂ = 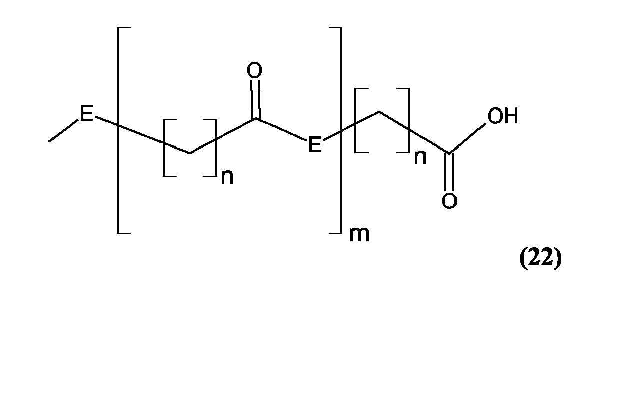 mit E = -O- oder -NR₅-,
n = 3 bis 15,
m=0 bis 10 und
R₅ = Wasserstoff oder Alkylgruppe
R₃ = R₁, R₂, -OR₆ oder -NR₇R₈
mit R₆, R₇ und R₈ = Wasserstoff, Alkyl- oder Arylgruppe, die jeweils substituiert oder unsubstituiert ist,
ist,

zeichnet sich dadurch aus, dass Cyanurchlorid mit von 0,5 bis 5 Moläquivalenten eines Amins der Formel (2)

H-A-B (2)

in Gegenwart einer Base und von 0,5 bis 5 Moläquivalenten einer Verbindung gemäß der Formel (3) 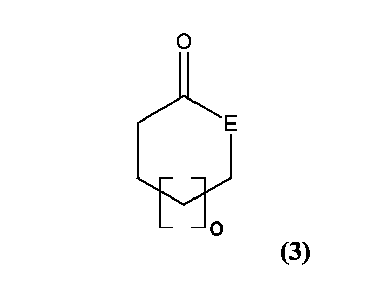 oder einer Verbindung gemäß der Formel (4) 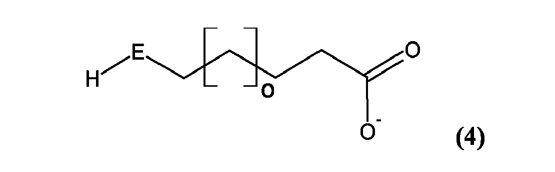 mit o von 0 bis 12,
E = -O- oder -NR₅- und
R₅ = Wasserstoff oder Alkylgruppe,
umgesetzt wird, wobei die Reihenfolge der beiden Reaktionsschritte beliebig ist.

In der Ausführungsform des erfindungsgemäßen Verfahrens, bei der Verbindungen mit R₃ ungleich R₁ oder R₂ hergestellt werden, werden in einem weiteren Reaktionsschritt 0,5 bis 5 Moläquivalenten einer Verbindung gemäß der Formel (16) oder (17) 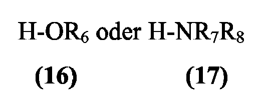 in Gegenwart einer Base umgesetzt.

Der Substituent vom Typ R₅ kann sowohl Wasserstoff als auch eine Alkylgruppe sein. Vorzugsweise ist der Substituent vom Typ R₅ Wasserstoff oder eine Alkylgruppe mit einer Kohlenstoffanzahl von 1 bis 16, bevorzugt von 1 bis 4. Diese Alkylgruppe des Substituenten vom Typ R₅ kann verzweigt oder unverzweigt sein, bevorzugt ist diese jedoch unverzweigt. Ferner ist diese Alkylgruppe des Substituenten vom Typ R₅ vorzugsweise unsubstituiert. Bevorzugt ist R₅ jedoch Wasserstoff. In den Verbindungen gemäß den Formeln (3) oder (4) ist o vorzugsweise von 0 bis 12, bevorzugt von 2 bis 8 und besonders bevorzugt 2.

Vorzugsweise werden in dem erfindungsgemäßen Verfahren als Verbindungen gemäß der Formel (3) Lactame oder Lactone eingesetzt, bevorzugt werden jedoch Lactame eingesetzt. Besonders bevorzugt wird Caprolactam in dem erfindungsgemäßen Verfahren eingesetzt. In einer besonderen Ausführungsform des erfmdungsgemäßen Verfahrens wird eine Verbindung gemäß der Formel (4) eingesetzt, besonders bevorzugt wird hierbei Natriumaminocapronat eingesetzt.

Es ist in dem erfmdungsgemäßen Verfahren auch möglich, dass das Edukt gemäß Formel (4) in-situ gebildet wird, beispielsweise durch den Einsatz von einer Verbindung gemäß Formel (3) und einer Base, insbesondere einem Alkalihydroxid, wie beispielsweise Natriumhydroxid. Vorzugsweise werden die beiden Edukte hierfür in einem Stoffmengenverhältnis von 5:1 bis 1:1, bevorzugt von 4:1 bis 1:1 besonders bevorzugt von 2:1 bis 1:1 eingesetzt.

In dem erfindungsgemäßen Verfahren werden von 0,5 bis 5, vorzugsweise von 1 bis 3 und bevorzugt von 1 bis 2 Moläquivalente der Verbindung gemäß der Formel (3) oder (4) bezogen auf das eingesetzte Cyanurchlorid eingesetzt.

Vorzugsweise werden in dem erfindungsgemäßen Verfahren als weiteres Edukt ein Amin gemäß der Formel (2) eingesetzt, wobei das Strukturfragment A sowohl -O- als auch -NR₄- sein kann. Bevorzugt wird ein Amin gemäß der Formel (2) eingesetzt, das als Strukturfragment A -NR₄- aufweist. Der Substituent vom Typ R₄ kann sowohl Wasserstoff als auch eine Alkylgruppe sein. Vorzugsweise ist der Substituent vom Typ R₄ Wasserstoff oder eine Alkylgruppe mit einer Kohlenstoffanzahl von 1 bis 10, bevorzugt von 2 bis 5. Diese Alkylgruppe des Strukturfragments A kann verzweigt oder unverzweigt sein, bevorzugt ist diese jedoch unverzweigt. Ferner ist diese Alkylgruppe vorzugsweise unsubstituiert. Besonders bevorzugt ist der Substituent vom Typ R₄ jedoch Wasserstoff.

In dem erfindungsgemäßen Verfahren wird ein Amin gemäß der Formel (2) eingesetzt, das als Substituent vom Typ B vorzugsweise einen Aminogruppe-haltiger Substituenten aufweist, wobei die Aminogruppe sich an einem aliphatischen Grundgerüst befinden kann oder die Aminogruppe ein aliphatisches cyclisches Amin sein kann. Bevorzugt weist dieser Substituent vom Typ B ein aliphatisches cyclisches Amin auf.

Bevorzugt werden in dem erfindungsgemäßen Verfahren Amine gemäß der Formel (2) eingesetzt, die einen Substituent vom Typ B gemäß der Formel (6) oder gemäß der Formel (5) aufweisen.

Die Substituenten vom Typ R₁₀ und R₁₁ können gleich oder verschieden sein und sind vorzugsweise Wasserstoff, eine Alkyl-, eine Cycloalkyl- oder eine Heterocycloalkylgruppe mit jeweils einer Kohlenstoffanzahl von 1 bis 20, bevorzugt von 2 bis 10 bzw. einer Anzahl an Kohlenstoffatomen und Heteroatomen von 1 bis 20, bevorzugt von 2 bis 10. Diese Alkylgruppe der Substituenten vom Typ R₁₀ und R₁₁ sind vorzugsweise verzweigt oder unverzweigt, bevorzugt sind diese jedoch unverzweigt. Ferner sind sie vorzugsweise unsubstituiert oder mit einer Aminogruppe substituiert, bevorzugt ist sie jedoch unsubstituiert. Die Cycloalkylgruppe der Substituenten vom Typ R₁₀ und R₁₁ ist vorzugsweise unsubstituiert oder substituiert, insbesondere ist diese Cycloalkylgruppe unsubstituiert. Die Heterocycloalkylgruppe der Substituenten vom Typ R₁₀ und R₁₁ ist vorzugsweise unsubstituiert oder substituiert, bevorzugt ist diese Heterocycloalkylgruppe mit ein oder mehreren Methylgruppen substituiert, bevorzugt ist sie eine Heterocycloalkylgruppe, die als Heteroatom ein oder mehrere Stickstoffatome aufweist, bevorzugt ist sie eine Heterocycloalkylgruppe gemäß der Formel (5).

Der Substituent vom Typ R₉ ist vorzugsweise Wasserstoff oder eine Alkylgruppe mit einer Kohlenstoffanzahl von 1 bis 16, bevorzugt von 1 bis 8 oder eine Alkoxygruppe mit einer verzweigten oder unverzweigten Alkylgruppe oder einer Cycloalkygruppe, besonders bevorzugt ist der Substituent vom Typ R₉ Wasserstoff.

Besonders bevorzugt werden in dem erfmdungsgemäßen Verfahren Amine mit einem Substituenten vom Typ B gemäß der Formel (6) eingesetzt. Besonders bevorzugt werden jedoch Amine mit einem Substituenten vom Typ B gemäß den Formeln (6a) oder (6b) eingesetzt.

Ganz besonders bevorzugt werden Amine gemäß der Formel (2a)

HNR₄-(CH₂)ₘ-NR₁₀R₁₁ (2a)

eingesetzt.

In einer besonders bevorzugten Ausführungsform des erfindungsgemäßen Verfahrens werden Amine eingesetzt, die einen Substituenten vom Typ B gemäß der Formel (5) aufweisen. Ganz besonders bevorzugt werden in dem erfindungsgemäßen Verfahren Amine gemäß Formel (2b): 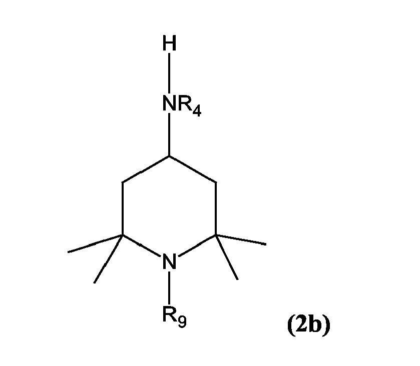 eingesetzt.

Es können in dem erfindungsgemäßen Verfahren auch Mischungen aus verschiedenen Verbindungen gemäß den Formeln (3) oder (4) bzw. auch Mischungen aus verschiedenen Aminen gemäß der Formel (2) eingesetzt werden.

In einer weiteren Ausführungsform des erfindungsgemäßen Verfahren wird als ein weiteres Edukt Verbindungen gemäß den Formeln (16) oder (17) eingesetzt, wobei R₆, R₇ und R₈ Wasserstoff, Alkyl- oder Arylgruppe ist, wobei die Alkyl- oder Arylgruppe unsubstituiert oder mit einem oder mehreren Substituenten der Formel R₆, R₇, R₈, -SO₃H oder -SO₃M, wobei M ein Alkalimetallkation, bevorzugt ein Lithium-, Natrium- oder Kaliumkation ist, substituiert ist. Die Alkyl- und/oder Arylgruppen der Substituenten vom Typ R₆, R₇ oder R₈ weisen bevorzugt von 1 bis 4, besonders bevorzugt von 1 bis 2 und ganz besonders bevorzugt einen Substituenten der Formel -SO₃H oder -SO₃M auf. Vorzugsweise sind die Substituenten vom Typ R₆, R₇ und R₈ Wasserstoff oder eine Alkylgruppe mit einer Kohlenstoffanzahl von 1 bis 18, bevorzugt von 2 bis 16. Diese Alkylgruppe des Substituenten vom Typ R₆, R₇ und R₈ kann verzweigt oder unverzweigt sein, bevorzugt ist diese jedoch unverzweigt. Die Arylgruppe der Substituenten vom Typ R₆, R₇ und R₈ ist vorzugsweise eine Phenylgruppe oder eine mit -SO₃H oder -SO₃M einfach substituierte Phenylgruppe, bevorzugt in para-Stellung. Die Substituenten vom Typ R₆, R₇ und R₈ können alle identisch, alle verschieden oder auch paarweise identisch sein. Bevorzugt sind die Substituenten vom Typ R₆, R₇ und R₈ Wasserstoff, Phenyl- oder eine mit -SO₃H oder -SO₃M in para-Stellung einfach substituierte Phenylgruppe.

Abhängig von der herzustellenden Triazinverbindung kann das erfindungsgemäße Verfahren aus zwei oder drei Reaktionsschritte für die eigentliche Umsetzung bzw. Reaktion bestehen.

In einer bevorzugten Ausführungsform des erfindungsgemäßen Verfahrens wird in einem ersten Reaktionsschritt Cyanurchlorid mit einem Amin gemäß der Formel (2) in Gegenwart einer Base in einem Lösemittel umgesetzt. Als Base wird vorzugsweise Natronlauge eingesetzt. Vorzugsweise wird hierbei ein Stoffmengenverhältnis von Amin zu Base von 1:1 eingesetzt. In diesem ersten Reaktionsschritt dieser bevorzugten Ausführungsform kann ein Lösemittel, ausgewählt aus Wasser, aromatischen Kohlenwasserstoffen, insbesondere Toluol, Xylol, Alkane, Ether, Ketone, wie beispielsweise Aceton, oder Ester eingesetzt werden, vorzugsweise wird Wasser als Lösemittel eingesetzt. Ungeeignet für diesen ersten Reaktionsschritt sind Alkohole, primäre oder sekundäre Amine als Lösemittel. In dem zweiten Reaktionsschritt erfolgt dann die Umsetzung mit einer Verbindung, ausgewählt aus Verbindungen gemäß Formel (3) oder (4).

In einer weiteren Ausführungsform des erfindungsgemäßen Verfahrens wird in einem ersten Reaktionsschritt Cyanurchlorid mit einer Verbindung, ausgewählt aus den Verbindungen gemäß der Formel (3) oder (4), und anschließend in einem weiteren Reaktionsschritt mit einem Amin gemäß Formel (2) umgesetzt. Die Lösemittel und das Stoffmengenverhältnis von Amin zu Base können analog zu der bevorzugten Ausführungsform gewählt werden.

Bei dem Reaktionsschritt mit einer Verbindung gemäß Formel (3) als Reaktant wird vorzugsweise eine Base bzgl. der Ringöffnung zugesetzt. Als Lösemittel werden hierbei insbesondere Wasser, Toluol, Xylol, Alkane, Ether, Ketone, wie beispielsweise Aceton, oder Ester, bevorzugt jedoch Wasser eingesetzt. In einer besonderen Ausführungsform des erfindungsgemäßen Verfahrens wird ein Lactam als Lösemittel eingesetzt, insbesondere wird hierbei eine Verbindung gemäß Formel (3) als Lösemittel eingesetzt, besonders bevorzugt wird als Lösemittel und Reaktant dieselbe Verbindung eingesetzt. Wird jedoch als Reaktant eine Verbindung gemäß der Formel (4) in einem Reaktionsschritt eingesetzt, so wird in Gegenwart eines Überschuss an der entsprechenden Verbindung gemäß Formel (3), dies bedeutet ein Stoffmengenverhältnis vorzugsweise von Verbindung gemäß der Formel (3) zum Cyanurchlorid von 1 : 4, insbesondere von 1,1 : 3,5, der Reaktionsschritt durchgeführt.

Beispielsweise wird beim Einsatz von Natriumaminocapronat als eine Verbindung gemäß Formel (4) als Reaktant die Reaktion in Gegenwart eines Überschusses an Caprolactam durchgeführt.

Dient das erfindungsgemäße Verfahren zur Herstellung von Triazinverbindungen gemäß Formel (1) mit R₃ = R₁ oder R₂, so wird in dem erfindungsgemäßen Verfahren in einem ersten Reaktionsschritt das Cyanurchlorid vorzugsweise mit von 0,5 bis 3, bevorzugt von 1 bis 2 Moläquivalenten bezogen auf die Stoffmenge an Cyanurchlorid an Reaktant A umgesetzt, wobei als Reaktant A entweder ein Amin gemäß Formel (2) oder eine Verbindung, ausgewählt aus Verbindungen gemäß Formel (3) oder (4), eingesetzt werden kann. In einem zweiten Reaktionsschritt des erfindungsgemäßen Verfahrens wird dann das erhaltene Zwischenprodukt mit von 0,5 bis 5, bevorzugt von 1 bis 4 Moläquivalenten bezogen auf die Stoffmenge an Cyanurchlorid an Reaktant B umgesetzt, wobei als Reaktant B
- eine Verbindung, ausgewählt aus Verbindungen gemäß Formel (3) oder (4), eingesetzt wird, wenn als Reaktant A ein Amin gemäß der Formel (2) eingesetzt wird, oder
- ein Amin gemäß Formel (2), wenn als Reaktant A eine Verbindung, ausgewählt aus Verbindungen gemäß Formel (3) oder (4),
   eingesetzt wird.
   Der erste Reaktionsschritt des erfindungsgemäßen Verfahrens wird vorzugsweise bei einer Temperatur von -20 bis 100°C, bevorzugt bei einer Temperatur von -10 bis 80°C und besonders bevorzugt von 0 bis 60°C durchgeführt. Der zweite Reaktionsschritt wird hingegen vorzugsweise bei einer Temperatur von 0 bis 200°C, bevorzugt bei 10 bis 180°C und besonders bevorzugt bei einer Temperatur von 20 bis 170°C durchgeführt. Bei einer Ausführungsform des erfindungsgemäßen Verfahrens, bei der im ersten Reaktionsschritt nur ein Chloratom des Cyanurchlorids mit dem Reaktanten A umgesetzt wird und in einem zweiten Reaktionsschritt die verbleibenden zwei Chloratome des Cyanurchlorids mit dem Reaktanten B umgesetzt wird, ist das Arbeiten mit einer Temperaturrampe in dem zweiten Reaktionsschritt von Vorteil.
   Der erste Reaktionsschritt des erfindungsgemäßen Verfahrens wird vorzugsweise bei einem Druck von 0,5 bis 1,5 bar, bevorzugt bei einem Druck von 0,8 bis 1,2 bar und besonders bevorzugt bei Atmosphärendruck durchgeführt. Der zweite Reaktionsschritt wird hingegen vorzugsweise bei einem Druck von 1 bis 11 bar, bevorzugt bei einem Druck von 1 bis 9 bar und besonders bevorzugt bei einem Druck von 1 bis 8 bar durchgeführt.
   In einer weiteren Ausführungsform des erfindungsgemäßen Verfahrens wird in einem ersten Reaktionsschritt Cyanurchlorid mit einer Verbindung, ausgewählt aus Verbindungen gemäß Formel (3) oder (4) in einem Lösemittel umgesetzt. In dem zweiten Reaktionsschritt erfolgt dann die Umsetzung mit einem Amin gemäß der Formel (2).
   In einer bevorzugten Ausführungsform des erfindungsgemäßen Verfahrens wird in dem ersten Reaktionsschritt das Cyanurchlorid mit von 1 bis 3, bevorzugt mit 2 Moläquivalenten bezogen auf die Stoffmenge an Cyanurchlorid an Reaktant A umgesetzt und in einem zweiten Reaktionsschritt wird dann das erhaltene Zwischenprodukt mit von 0,5 bis 5, bevorzugt von 1 bis 3 Moläquivalenten bezogen auf die Stoffmenge an Cyanurchlorid an Reaktant B umgesetzt. Hierbei wird der erste Reaktionsschritt vorzugsweise bei einer Temperatur von 0 bis 100°C, bevorzugt bei einer Temperatur von 10 bis 80°C und besonders bevorzugt von 20 bis 60°C durchgeführt. Der zweite Reaktionsschritt wird hingegen vorzugsweise bei einer Temperatur von 80 bis 200°C, bevorzugt bei 90 bis 180°C und besonders bevorzugt bei einer Temperatur von 100 bis 170°C durchgeführt.
   Dient das erfindungsgemäße Verfahren zur Herstellung von Triazinverbindungen gemäß Formel (1) mit R₃ = -OR₆ oder -NR₇R₈, so wird in dem erfindungsgemäßen Verfahren in einem ersten Reaktionsschritt das Cyanurchlorid mit von 0,5 bis 2 Moläquivalenten, bevorzugt mit 1 Moläquivalent bezogen auf die Stoffmenge an Cyanurchlorid an Reaktant A umgesetzt, wobei als Reaktant A entweder ein Amin gemäß Formel (2), eine Verbindung, ausgewählt aus Verbindungen gemäß Formel (3) oder (4), oder eine Hydroxy- oder Aminoverbindung der Formeln (16) oder (17) eingesetzt werden kann. In einem zweiten Reaktionsschritt des erfindungsgemäßen Verfahrens wird dann das erhaltene Zwischenprodukt mit von 0,5 bis 2, bevorzugt mit 1 Moläquivalenten bezogen auf die Stoffmenge an Cyanurchlorid an Reaktant B umgesetzt, wobei als Reaktant B
- eine Verbindung, ausgewählt aus Verbindungen gemäß Formeln (3), (4), (16) oder (17) eingesetzt wird, wenn als Reaktant A eine Verbindung der Formel (2) eingesetzt wird, oder
- eine Verbindung, ausgewählt aus Verbindungen gemäß Formel (2), (16) oder (17) eingesetzt wird, wenn als Reaktant A eine Verbindung, ausgewählt aus Verbindungen gemäß Formel (3) oder (4), eingesetzt wird, oder
- eine Verbindung, ausgewählt aus Verbindungen gemäß Formel (2), (3) oder (4) eingesetzt wird, wenn als Reaktant A eine Verbindung, ausgewählt aus Verbindungen gemäß Formel (16) oder (17), eingesetzt wird.

In einem dritten Reaktionsschritt dieser besonderen Ausführungsform des erfindungsgemäßen Verfahrens wird dann das erhaltene Zwischenprodukt mit von 0,5 bis 2, bevorzugt mit 1 Moläquivalenten bezogen auf die Stoffmenge an Cyanurchlorid an Reaktant C umgesetzt, wobei als Reaktant C
- eine Verbindung, ausgewählt aus Verbindungen gemäß Formeln (16) oder (17), eingesetzt wird, wenn als Reaktanten A und B Verbindungen der Formel (2) und (3) oder (4) eingesetzt wird, oder
- eine Verbindung, ausgewählt aus Verbindungen gemäß Formeln (3) oder (4), eingesetzt wird, wenn als Reaktanten A und B Verbindungen der Formeln (3) oder (4) und (16) oder (17) eingesetzt wird, oder
- eine Verbindung gemäß Formel (2) eingesetzt wird, wenn als Reaktanten A und B Verbindungen der Formel (3) oder (4) und (16) oder (17) eingesetzt wird.

Der erste Reaktionsschritt dieser besonderen Ausführungsform des erfmdungsgemäßen Verfahrens wird vorzugsweise bei einer Temperatur von -20 bis 80 °C, bevorzugt bei einer Temperatur von -10 bis 60°C und besonders bevorzugt von 0 bis 40°C durchgeführt. Der zweite Reaktionsschritt wird hingegen vorzugsweise bei einer Temperatur von 0 bis 100°C, bevorzugt bei 10 bis 80°C und besonders bevorzugt bei einer Temperatur von 20 bis 60°C durchgeführt. Der dritte Reaktionsschritt wird vorzugsweise bei einer Temperatur von 80 bis 200°C, bevorzugt bei 90 bis 180°C und besonders bevorzugt bei einer Temperatur von 100 bis 170°C durchgeführt

Der erste und der zweite Reaktionsschritt dieser besonderen Ausführungsform des erfindungsgemäßen Verfahrens wird vorzugsweise bei einem Druck von 0,5 bis 1,5 bar, bevorzugt bei einem Druck von 0,8 bis 1,2 bar und besonders bevorzugt bei Atmosphärendruck durchgeführt. Der dritte Reaktionsschritt wird hingegen vorzugsweise bei einem Druck von 1 bis 11 bar, bevorzugt bei einem Druck von 1 bis 9 bar und besonders bevorzugt bei einem Druck von 1 bis 8 bar durchgeführt.

Die einzelnen Reaktionsschritte des erfindungsgemäßen Verfahrens können in jeweils einer Verfahrensstufe des erfindungsgemäßen Verfahrens durchgeführt werden, wobei die jeweils entstehenden Zwischenprodukte abgetrennt und isoliert werden können und somit als Edukt für die darauf folgende Verfahrensstufe eingesetzt werden können.

In einer besonderen Ausführungsform werden die Zwischenprodukte der jeweiligen Verfahrensstufe nicht abgetrennt und isoliert - mit Ausnahme der letzten Verfahrensstufe - sondern direkt der nächsten Verfahrenstufe als Edukt zugeführt. In dieser Ausführungsform des erfmdungsgemäßen Verfahren erfolgt die Bildung der Zwischenprodukte somit "in situ".

In einer weiteren Ausführungsform des erfindungsgemäßen Verfahrens werden alle Reaktionsschritte in einem Reaktionsapparat, insbesondere in einem Autoklaven, durchgeführt. Hierbei kann die Umsetzung der Verbindungen gemäß Formel (3) mit einer Base in einer separaten Verfahrensstufe bzw. in einem separaten Reaktionsgefäß erfolgen. Die Umsetzung der Verbindungen gemäß Formel (3) mit einer Base kann jedoch auch in demselben Reaktionsgefäß erfolgen, so dass alle Reaktionsschritte des erfindungsgemäßen Verfahrens in demselben Reaktionsgefäß erfolgen. Auf diese Weise können alle drei Substitutionen am Triazinring des erfindungsgemäßen Verfahrens in einem Reaktionsgefäß durchgeführt werden.

Generell kann die Umsetzung der Verbindungen gemäß Formel (3) mit einer Base in einer separaten Verfahrensstufe bzw. in einem separaten Reaktionsgefäß erfolgen. Die Umsetzung der Verbindungen gemäß Formel (3) mit einer Base kann jedoch auch in demselben Reaktionsgefäß, in dem auch die jeweilige Reaktionsstufe gerade erfolgt.

Die Aufarbeitung des Reaktionsgemisches dient überwiegend dazu, Natriumchlorid als Reaktionsnebenprodukt abzutrennen. Bei den wasserunlöslichen erfindungsgemäßen Triazinverbindungen wird das Natriumchlorid in Wasser gelöst und durch Filtration der wässrigen Suspensionen und anschließendes Waschen des Filterkuchens oder durch Extraktion des Zielproduktes mit einem organischen Lösemittel, vorzugsweise dem während der Reaktion eingesetzten organischen Lösemittels entfernt. Bei den wasserlöslichen Triazinverbindungen erfolgt die Abtrennung des ebenfalls wasserlöslichen Natriumchlorids vorzugsweise mittels Elektrodialyse über Membrane oder mittels Ionenaustauschchromatographie, bevorzugt erfolgt die Abtrennung des Natriumchlorids mittels Ionenaustauschchromatographie.

Das auf diese Weise aufgearbeitete als Lösung in Wasser oder in einem organischen Lösemittel vorliegenden Reaktionsgemisch kann anschließend direkt eingesetzt werden, in einer besonderen Ausführungsform erfolgt vor dem Einsatz als Stabilisator eine Trocknung.

Die nach den einzelnen Reaktionsschritten auftretenden Zwischenprodukte können in einer besonderen Ausführungsform des erfindungsgemäßen Verfahrens aus der Reaktionsmischung isoliert und gereinigt werden. Vorzugsweise erfolgt dies durch Kristallisation, Filtration und gegebenenfalls einer Wäsche aus der Reaktionsmischung. Die Isolierung und Reinigung dieser Zwischenprodukte kann auch mittels einer Extraktion mit einem organischen Lösemittel erfolgen, bevorzugt mit dem organischen Lösemittel, das bereits für die Reaktion eingesetzt worden ist. Die auf diese Weise isolierten und gereinigten Zwischenprodukte, die in der Regel als Feststoffe vorliegen, können dann in dem nächsten Reaktionsschritt des erfindungsgemäßen Verfahrens eingesetzt werden.

In einer weiteren Ausführungsform des erfmdungsgemäßen Verfahrens erfolgt keine Isolierung und Aufarbeitung dieser Zwischenprodukte. Die Reaktionsschritte werden hierbei nacheinander ausgeführt, ohne dass die Zwischenprodukte isoliert und aufgearbeitet werden.

Die erfmdungsgemäßen Zusammensetzungen weisen zumindest zwei verschiedene Triazinverbindungen gemäß der Formel (1) auf.

Vorzugsweise weist die erfindungsgemäße Zusammensetzung
- 85 bis 95 Gew.-% an erfindungsgemäßen Triazinverbindungen gemäß der Formel (18), 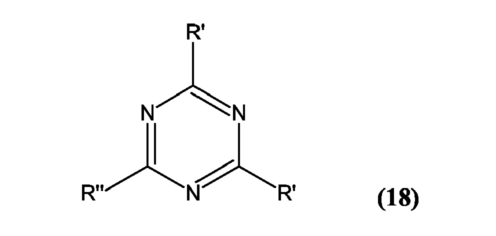
- 0 bis 10 Gew.-% an Triazinverbindungen gemäß Formel (19), 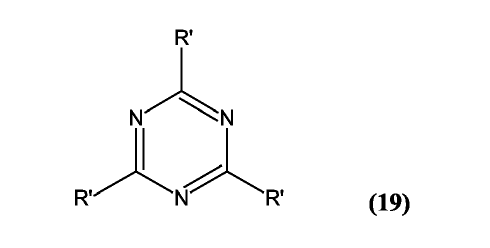
- 0 bis 10 Gew.-% an Triazinverbindungen gemäß Formel (20), 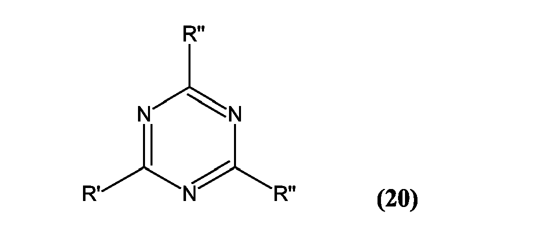 auf, wobei R' = R₁ oder R₂ und R'' = R₂ oder R₁ und R' nicht identisch ist mit R'', insbesondere bedeutet dies, dass wenn R' = R₁ ist, so ist R'' = R₂ bzw. umgekehrt.
   In einer bevorzugten Ausführungsform der erfindungsgemäßen Zusammensetzung ist R' = R₁ und R" = R₂, diese Zusammensetzung kann erhalten werden, wenn die Edukte Cyanurchlorid, Amin der Formel (2) und eine Verbindung gemäß der Formel (3) oder (4) in einem Stoffmengenverhältnis von 1:2:1 in dem erfindungsgemäßen Verfahren eingesetzt werden.
   In einer besonders bevorzugten Ausführungsform der erfindungsgemäßen Zusammensetzung ist R' = R₂ und R" = R₁, diese Zusammensetzung kann erhalten werden, wenn die Edukte Cyanurchlorid, Amin der Formel (2) und eine Verbindung gemäß der Formel (3) oder (4) in einem Stoffmengenverhältnis von 1:1:2 in dem erfindungsgemäßen Verfahren eingesetzt werden.
   In einer weiteren Ausführungsform der erfindungsgemäßen Zusammensetzung weist diese
- 30 bis 95 Gew.-% an erfindungsgemäßen Triazinverbindungen gemäß der Formel (18), 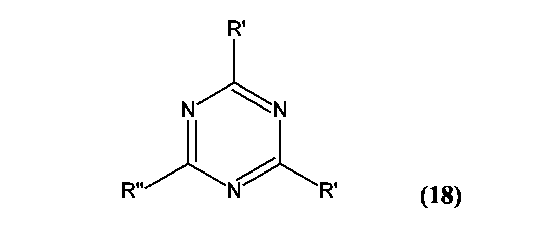
- 0 bis 60 Gew.-% an erfindungsgemäßen Triazinverbindungen gemäß Formel (20), 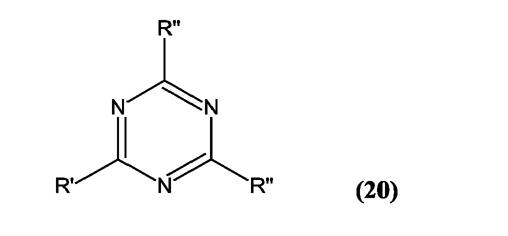
- 0 bis 10 Gew.-% an Triazinverbindungen gemäß Formel (19) und (21), 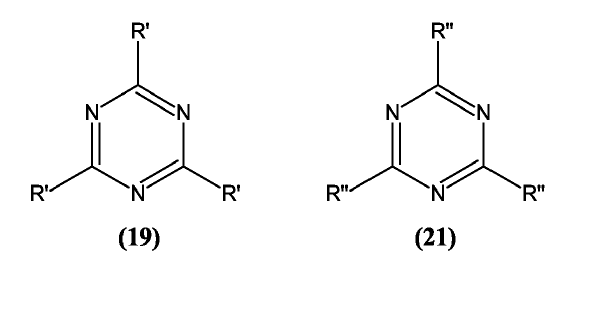 auf, wobei R' = R₁ oder R₂ und R" = R₂ oder R₁ und R' nicht identisch ist mit R'', insbesondere bedeutet dies, dass wenn R' = R₁ ist, so ist R" = R₂ bzw. umgekehrt. Diese Zusammensetzung kann erhalten werden, wenn die Edukte Cyanurchlorid, Amin der Formel (2) und eine Verbindung gemäß der Formel (3) oder (4) in einem Stoffmengenverhältnis von 1:1,5:1,5 in dem erfindungsgemäßen Verfahren eingesetzt werden.
   In einer weiteren bevorzugten Ausführungsform der erfindungsgemäßen Zusammensetzung weist diese
- 60 bis 90 Gew.-% an erfindungsgemäßen Triazinverbindungen gemäß der Formel (23), 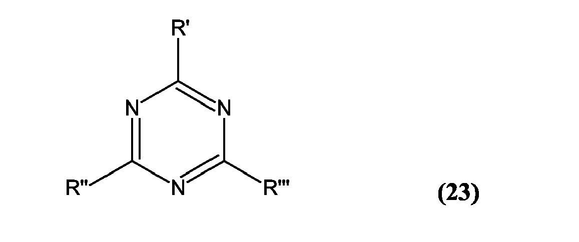
- 1 bis 30 Gew.-% an erfindungsgemäßen Triazinverbindungen mit jeweils zwei Substituenten vom gleichen Typ und einem davon verschiedenen dritten Substituent, ausgewählt aus R', R" oder R"', beispielsweise Triazinverbindungen gemäß Formel (18) oder (20),
- 0,5 bis 10 Gew.-% an Triazinverbindungen, wobei alle drei Substituenten vom gleichen Typ, ausgewählt aus R', R" oder R"', sind, beispielsweise Triazinverbindungen gemäß Formel (19) oder (21),
   auf, wobei R' = R₁, R₂ oder R₃, R" = R₂, R₁ oder R₃ und R"' = R₃, R₂ oder R₁ und R', R" und R"' nicht identisch sind, insbesondere bedeutet dies, dass wenn R' = R₁ ist, so ist R'' = R₂ und R"' = R₃ bzw. umgekehrt. Diese Zusammensetzung kann erhalten werden, wenn die Edukte Cyanurchlorid, Amin der Formel (2), eine Verbindung gemäß der Formel (3) oder (4) und eine Verbindung gemäß der Formel (16) oder (17) in einem Stoffmengenverhältnis von 1 : (von 0,5 bis 2) : (von 0,5 bis 2) : (von 0,5 bis 5), vorzugsweise in einem Stoffmengenverhältnis von 1 : 1 : 1 : (von 1 bis 4) in dem erfindungsgemäßen Verfahren eingesetzt werden.

Die erfindungsgemäße Lösung weist zumindest eine Triazinverbindung gemäß der Formel (1) auf, vorzugsweise weist diese von 1 bis 50 Gew.%, bevorzugt von 20 bis 45 Gew.-% an Triazinverbindungen gemäß der Formel (1) auf. Vorzugsweise weist die erfindungsgemäße Lösung als Lösemittel Wasser, ein organisches Lösemittel, ausgewählt aus aromatischen Kohlenwasserstoffen, insbesondere Toluol, Xylol, Alkane, Ether, Ketone, wie beispielsweise Aceton, oder Ester eingesetzt werden, oder die für die Herstellung der erfindungsgemäßen Triazinverbindungen eingesetzte Verbindung gemäß der Formel (3) auf. Bevorzugt weist die erfindungsgemäße Lösung als Lösemittel Wasser auf.

Die erfindungsgemäße Verwendung der Triazinverbindungen gemäß Formel (1) erfolgt zur Stabilisierung von Polymeren.

Insbesondere können die erfindungsgemäßen Triazinverbindungen zur Stabilisierung von Polyamiden eingesetzt werden. Als Polyamide kommen in erster Linie aliphatische Homo- und Copolykondensate in Frage, beispielsweise PA 46, PA 66, PA 68, PA 610, PA 612, PA 410, PA 810, PA 1010, PA 412, PA 1012, PA 1212, PA6, PA 7, PA 8, PA 9, PA 10, PA 11 und PA 12. Bevorzugt werden die erfindungsgemäßen Triazinverbindungen zur Stabilisierung von PA 410, PA 810, PA 1010, PA 412, PA 1012, PA 1212, PA 6, PA 7, PA 8, PA 9, PA 10, PA 11 und PA 12 eingesetzt. Diese Kennzeichnung der Polyamide entspricht internationaler Norm, wobei die erste(n) Ziffer(n) die C-Atomzahl des Ausgangsdiamins und die letzte(n) Ziffer(n) die C-Atomzahl der Dicarbonsäure angeben. Wird nur eine Zahl genannt, so bedeutet dies, dass von einer α,ω-Aminocarbonsäure bzw. von dem davon abgeleiteten Lactam ausgegangen worden ist. Im Übrigen sei verwiesen auf H. Domininghaus, "Die Kunststoffe und ihre Eigenschaften", Seiten 272 ff., VDI-Verlag, 1976.

Weiterhin können die erfindungsgemäßen Triazinverbindungen zur Stabilisierung von Polyester eingesetzt werden. Insbesondere können diese zur Stabilisierung von Polyestern eingesetzt werden, die durch Polykondensation von Diolen mit Dicarbonsäure bzw. deren polyesterbildenden Derivaten wie Dimethylestern hergestellt wurden. Geeignete Diole haben die Formel HO-R-OH, wobei R einen divalenten, verzweigten oder unverzweigten aliphatischen und/oder cycloaliphatischen Rest mit 2 bis 18, vorzugsweise 2 bis 12, C-Atomen darstellt. Geeignete Dicarbonsäuren haben die Formel HOOC-R'-COOH, wobei R' einen divalenten aliphatischen, cycloaliphatische oder aromatischen Rest mit 2 bis 18, vorzugsweise 4 bis 12, C-Atomen bedeutet. Die Herstellung dieser Polyester gehört zum Stand der Technik (DE-OSS 24 07 155, 24 07 156; Ullmanns Encyclopädie der technischen Chemie, 4. Aufl., Bd. 19, Seiten 65 ff., Verlag Chemie, Weinheim, 1980).

Die erfmdungsgemäßen Triazinverbindungen können bereits bei der Polykondensation zugesetzt werden, dies hat den Vorteil, dass die so hergestellten Polyamide die Additive, welche die mechanische Festigkeit, die Stabilität gegen oxidativen und lichtinduzierten Abbau und die Anfärbbarkeit verbessern, kovalent und damit permanent gebunden enthalten. Die Zugabe der erfmdungsgemäßen Triazinverbindungen zur Polykondensation erfolgt hierbei so, dass die erfindungsgemäßen Triazinverbindungen mittels ihrer funktionellen Gruppen in die Polymerkette eingebunden werden können.
Die nachfolgenden Beispiele sollen das erfindungsgemäße Verfahren sowie die erfindungsgemäßen Triazinverbindungen näher erläutern, ohne dass die Erfindung auf diese Ausführungsform beschränkt sein soll.

### Beispiel 1 Herstellung von N-[4,6-Bis-(N-[2,2,6,6-tetramethyl-4-piperidyl-amino])-1,3,5-triazin-2-yl]-6-aminohexansäure

### 1.1 Herstellung des Zwischenprodukts 2-Chlor-4,6-bis-(N-[2,2,6,6-tetramethyl-4-piperidylamino])-1,3,5-triazin

In einem 6-1-Glasreaktionskolben wurden 276,6 g Cyanurchlorid (1,5 mol) in 3 1 Wasser bei 5 °C vorgelegt. Nach Zugabe von 468,9 g 4-Amino-2,2,6,6-tetramethylpiperidin (3,0 mol) bei 5 bis 20 °C und 480 g einer 25 Gew.%igen Natronlauge (entspricht 3,0 mol Natriumhydroxid) bei 20 bis 25 °C wurde eine Stunde bei 25 °C und eine Stunde bei 60 °C gerührt. Nach Abkühlen auf 25 °C wurde der erhaltene Feststoff abfiltriert und dreimal mit je 2 1 Wasser gewaschen und 20 Stunden bei 15 mbar und 100 °C getrocknet. Die Ausbeute betrug 597,0 g (1,41 mol, 94 % bezogen auf die eingesetzte Menge an Cyanurchlorid).

### 1.2 Herstellung des Rohprodukts

In einem 2-1-Glasreaktionskolben wurden 565,8 g ε-Caprolactam (5,0 mol) vorgelegt und bei 80 °C geschmolzen. Nach Zugabe von 160 g einer 25 Gew.-%igen Natronlauge (entspricht 1,0 mol Natriumhydroxid) wurde 2 Stunden bei 126 bis 130 °C unter Rühren erhitzt. Zu dieser geschmolzenen Lösung von Natriumaminocapronat in ε-Caprolactam wurde bei 110 °C 424,0g des Zwischenprodukts hergestellt gemäß Beispiel 1.1 (1 mol) zugegeben. Diese Suspension wurde 3 Stunden bei atmosphärischem Druck bei 126 bis 135 °C unter Rückfluss erhitzt. Nach dem Abkühlen auf 80 °C wurden 800 ml Essigsäureethylester zur Kristallisation des Rohproduktes zugesetzt. Der Feststoff wurde nach dem Abkühlen auf 40 °C abfiltriert, zur Entfernung des überschüssigen ε-Caprolactams wurde zweimal mit je 400 ml Essigsäureethylester gewaschen und bei 15 mbar und 50 °C getrocknet. Die Ausbeute betrug 545,2 g.

### 1.3 Aufarbeitung des Rohprodukts

### a.) mittels Elektrodialyse:

60,8 g des Rohproduktes (aus Beispiel 1.2) wurden in 547 g Wasser gelöst. Die so hergestellte ca. 10 Gew.-%ige Lösung wurde in einer Elektrodialyseapparatur (3 Kreislauf-Stapelaufbau / Platinanode, VA-Stahl-Kathode / Anodentauschermembran AHA 1, Kathodentauschermembran C 22 / Anoden-Kathodenkreislösung: 5 %ige wässrige Natriumsulfatlösung, Salzaufnehmerkreislösung: 3 %ige wässrige Natriumchloridlösung / Spannung 6 V) 24 Stunden bei 25 °C und einer Stromstärke von 1,96 bis 0,0 A entsalzt. Die Produktlösung wurde bei 130 °C und Normaldruck eingedampft und der Feststoff danach bei 90 °C und 15 mbar getrocknet. Die Ausbeute betrug 54,2 g (0,10 mol, 86 % bezogen auf die eingesetzte Menge an Zwischenprodukt aus 1.1).

Analyse des Produkts mittels HPLC (CLND):

| **Zusammensetzung des Produkts** | **[in Gew.-%]** |
|---|---|
| Erfindungsgemäße Triazinverbindung (gemäß Formel (1) mit R₁ gemäß Formel (7b) und R₂ mit E = -NH- und n = 5) | 92,1 |
| *R₂ mit m = 0; R₃ = R₁* | *83,3* |
| *R₂ mit m* = *1-3; R₃ = R₁* | *8,1* |
| *R₂ mit m = 0; R₃ = -OH* | *0,7* |
| ε-Caprolactam | 7,9 |

Die Ermittlung der Zusammensetzung der Produkte erfolgte mittels HPLC und einem CLND-Detektor (Chemiluminescence Nitrogen Detector). Dieser Art von Detektor ermöglicht die äquimolare Detektion von stickstoffhaltigen Verbindungen.

### b) mittels Ionenaustauschchromatographie:

Die Chromatographie erfolgte in einer Glassäule (1200 ml, Höhe 40 cm, Durchmesser 6,2 cm) bei 25 °C. Als Ionentauscherharz wurde Amberlyst 35 der Fa. Rohm and Haas (1,9 eq. H⁺/1, 5,2 eq. H⁺/kg) eingesetzt. Als Lösemittel wurde entsalztes Wasser verwendet. Die Kontrolle der Eluenten erfolgte per Messung der pH-Werte und der Brechungsindices. 1060 ml (2,0 mol H⁺-Äquivalente) des stark sauren Ionentauscherharzes wurden in der H⁺-Form vorgelegt. Nach Zugabe von 314,4 g an Rohprodukt aus Beispiel 1.2 in Form einer 25 Gew.-% igen wässrigen Lösung wurde mit Wasser so lange gespült bis ein pH-Wert von 6 bis 7 erreicht wurde. Das Produkt wurde mit 360 ml einer 14 Gew.-%iger wässriger NH₄OH-Lösung eluiert. Der Ionentauscher wurde nach der Spülung mit deionisiertem Wasser bis zu einem pH-Wert 7-8 mit 10 %iger Schwefelsäure regeneriert. Das gesamte Eluat mit dem Produkt wurde bei 40 °C und 30 mbar eingedampft und der erhaltene Feststoff bei 15 mbar und 100 °C getrocknet. Die Ausbeute betrug 258,7 g (0,50 mol, 87 % bezogen auf die eingesetzte Menge an Zwischenprodukt aus 1.1).

Analyse des Produkts mittels HPLC (CLND):

| **Zusammensetzung des Produkts** | **[in Gew.-%]** |
|---|---|
| Erfindungsgemäße Triazinverbindung (gemäß Formel (1) mit R₁ gemäß Formel (7b) und R₂ mit E = -NH- und n = 5) | 100,0 |
| *R₂ mit m = 0; R₃ = R₁* | *91,0* |
| *R₂ mit m* = *1-3*; *R₃* = *R₁* | *8,3* |
| *R₂ mit m = 0; R₃ = -OH* | *0,7* |

### Beispiel 2 Herstellung von N-[4,6-Bis-(N-[3-(diethylamino)-1-propyl-amino])-1,3,5-triazin-2-yl]-6-aminohexansäure

### 2.1 Herstellung des Zwischenprodukts 6-Chlor-2,4-bis(N-[3-(diethylamino)-1-propylamino])-1,3,5-triazin

276,6 g Cyanurchlorid (1,5 mol) wurden in 3 1 Wasser bei 5 bis 20 °C zuerst mit 390,7 g 3-(Diethylamino)-1-propylamin (3,0 mol) und danach bei 20 °C mit 480,0 g 25 Gew.-%iger Natronlauge (entspricht 3,0 mol Natriumhydroxid) umgesetzt und eine Stunde bei 40 °C gerührt. Der Feststoff wurde bei 25 °C abfiltriert, zweimal mit je 1 l Wasser gewaschen und bei 15 mbar und 80 °C 20 Stunden getrocknet. Die Ausbeute betrug 457,5 g (1,23 mol, 82 % bezogen auf die eingesetzte Menge an Cyanurchlorid).

### 2.2 Herstellung des Rohprodukts

Analog zu Beispiel 1.2 wurde eine bei 110°C geschmolzene Lösung von Natriumaminocapronat (1,0 mol) in ε-Caprolactam (4 mol) hergestellt. Nach Zugabe von 371,9 g des Zwischenprodukts hergestellt gemäß Beispiel 2.1 (1,0 mol) wurde 3 Stunden bei atmosphärischem Druck und 125 bis 133 °C unter Rückfluss erhitzt. Nach Kristallisation, Filtration, Wäsche mittels Essigsäureethylester und Trocknung analog zu Beispiel 1.2 erhält man 499,1 g des Rohprodukts.

### 2.3 Aufarbeitung des Rohprodukts

Analog zu Beispiel 1.3 b.) wurden 290,1 g des Rohprodukts aus Beispiel 2.2 via Ionenaustauscherchromatographie entsalzt und gereinigt. Die Ausbeute betrug 232,8 g (0,50 mol, 86 % bezogen auf die eingesetzte Menge an Zwischenprodukt aus Beispiel 2.1).

**Analyse des Produkts mittels HPLC (CLND):**

| **Zusammensetzung des Produkts** | **[in Gew.-%]** |
|---|---|
| Erfindungsgemäße Triazinverbindung (gemäß Formel (1) mit R₁, R₃ gemäß Formel (7c) und R₂ mit E = -NH-, n = 5) | 100,0 |
| *R₂ mit m = 0; R₃ = R₁* | *93,8* |
| *R₂ mit m* = *1-3; R₃* = *R₁* | *5,4* |
| *R₂ mit m* = *0; R₃* = *-OH* | *0,8* |

### Beispiel 3 Herstellung von N-[4,6-Bis-(N-[2,2,6,6-tetramethyl-4-piperidyl]-N-butylamino)-1,3,5-triazin-2-yl]-6-aminohexansäure

### 3.1 Herstellung des Zwischenprodukts 6-Chlor-2,4-bis-(N-[2,2,6,6-tetramethyl-4-piperidyl]-N-butylamino)-1,3,5-triazin

In einem 6-1-Glasreaktionskolben wurden 276,6 g Cyanurchlorid (1,5 mol) in 3 1 Wasser bei 5 °C vorgelegt. Nach Zugabe von 637,2 g 4-N-Butylamino-2,2,6,6-tetramethylpiperidin (3 mol) bei 5 bis 20 °C und 480 g einer 25 Gew.-%igen Natronlauge (entspricht 3,0 mol Natriumhydroxid) bei 20 bis 25 °C wurde eine Stunde bei 25 °C und eine Stunde bei 60 °C gerührt. Nach Abkühlen auf 25 °C wurde der erhaltene Feststoff abfiltriert und dreimal mit je 21 Wasser gewaschen und 20 Stunden bei 15 mbar und 100 °C getrocknet. Die Ausbeute betrug 773,8 g (1,44 mol, 96 % bezogen auf die eingesetzte Menge an Cyanurchlorid).

### 3.2 Herstellung des Rohprodukts

Analog zu Beispiel 1.2 wurde eine bei 110 °C geschmolzene Lösung von Natriumaminocapronat (1,0 mol) in ε-Caprolactam (4 mol) hergestellt. Nach Zugabe von 536,3 g des Zwischenprodukts hergestellt gemäß Beispiel 3.1 (1,0 mol) wurde 3 Stunden bei atmosphärischem Druck und 128 bis 136 °C unter Rückfluss erhitzt. Nach Kristallisation, Filtration, Wäsche mittels Essigsäureethylester und Trocknung analog zu Beispiel 1.2 erhält man 670,8 g an Rohprodukt (91 % bezogen auf die eingesetzte Menge an Zwischenprodukt aus Beispiel 3.1).

### 3.3 Aufarbeitung des Rohprodukts

Analog zu Beispiel 1.3 b.) wurden 367,7 g an Rohprodukt via Ionenaustauscherchromatographie entsalzt und gereinigt. Die Ausbeute betrug 313,0 g (0,50 mol, 90 % bezogen auf die eingesetzte Menge an Zwischenprodukt aus Beispiel 3.1).

**Analyse des Produkts mittels HPLC (CLND):**

| **Zusammensetzung des Produkts** | **[in Gew.-%]** |
|---|---|
| Erfindungsgemäße Triazinverbindung (gemäß Formel (1) mit R₁, R₃ gemäß Formel (7a) und R₂ mit E = -NH-, n = 5) | 100,0 |
| *R₂ mit m = 0; R₃ = R₁* | *92,4* |
| *R₂ mit m* = *1-3; R₃* = *R₁* | *7,0* |
| *R₂ mit m = 0; R₃ = -OH* | *0,6* |

### Beispiel 4 Herstellung von N,N'-[6-(N-[2,2,6,6-Tetramethyl-4-piperidyl-aminol)-1,3,5-triazin-2,4-diyl]-bis-(6-aminohexansäure)

### 4.1 Herstellung des Zwischenprodukts N,N'-[6-Chlor-1,3,5-triazin-2,4-diyl]-bis-(6-aminohexansäure)

In einem 4-1-Glasreaktionskolben wurden 452,6 g ε-Caprolactam (4,0 mol) mit 640 g 25 Gew.-%iger Natronlauge (entspricht 4,0 mol Natriumhydroxid) bei 120 bis 126 °C eine Stunde lang umgesetzt. Die Natriumaminocapronatlösung wurde mit 1500 ml Wasser verdünnt, auf 5 °C abgekühlt und mit 368,8 g Cyanurchlorid (2,0 mol) bei 5 bis 12 °C umgesetzt und eine Stunde lang bei 20-25 °C gerührt. Der Feststoff wurde bei 25 °C abfiltriert, zweimal mit je 1 1 Wasser gewaschen und bei 15 mbar und 80 °C 20 Stunden lang getrocknet. Die Ausbeute betrug 593,9 g (1,54 mol, 77 % bezogen auf die eingesetzte Menge an Cyanurchlorid)

**Analyse des Zwischenproduktes mittels HPLC (CLND):**

| **Zusammensetzung des Zwischenprodukts** | **[in Gew.-%]** |
|---|---|
| Triazinverbindung (analog zu Formel (1) mit R₁ = Cl und R₂ mit E = -NH- und n = 5) | 96,4 |
| *R₂ mit m = 0;* *R₃ = R₂, wobei E = -NH- , n* = *5 und m = 0* | *85,0* |
| *R₂ mit m = 1-3;* *R₃* = *R₂, wobei E = -NH-, n = 5 und m = 0-3* | *10,0* |
| *R₂ mit m = 0-1;* *R₃ = -OH*- | *1,4* |
| Aminocapronsäure | 3,6 |

### 4.2 Herstellung des Rohprodukts

186,9 g des Zwischenprodukts hergestellt gemäß Beispiel 4.1 (0,5 mol) wurden in einem 2-1-Glasreaktionskolben in 11 Wasser mit 78,2 g 4-Amino-2,2,6,6-tetramethylpiperidin (0,5 mol) bei 25 bis 40 °C und mit 80,0 g einer 25 Gew.-%igen Natronlauge (entspricht 0,5 mol Natriumhydroxid) bei 35 bis 40 °C umgesetzt und 3 Stunden lang bei 102 bis 104 °C erhitzt. Nach Abkühlen und Entspannen wurden 1345 g Filtrat erhalten.

### 4.3 Aufarbeitung des Rohprodukts

Analog zu Beispiel 1.3 b.) wurde das Filtrat über 1000 ml Amberlyst 35 (1,9 mol H⁺) entsalzt und gereinigt. Die Ausbeute betrug 235,8 g (0,48 mol, 96 % bezogen auf die eingesetzte Menge an Zwischenprodukt 4.1).

**Analyse des Produkts mittels HPLC (CLND):**

| **Zusammensetzung des Produkts** | **[in Gew.-%]** |
|---|---|
| Erfindungsgemäße Triazinverbindung (gemäß Formel (1) mit R₁ gemäß Formel (7b) und R₂ mit E = -NH- und n = 5) | 100,0 |
| *R₂ mit m = 0;* *R₃* = *R₂* , *wobei E* = *-NH-, n = 5 und m = 0* | *89,8* |
| *R₂ mit m = 1-3;* *R₃* = *R*_{2,} *wobei E = -NH-, n = 5 und m = 0-3* | *8,9* |
| *R₂ mit m = 0-1;* *R₃ = -OH* | *1,3* |

### Beispiel 5 Herstellung von N,N'-[6-(N-[3-(diethylamino)-1-propyl-amino])-1,3,5-triazin-2,4-diyl]-bis-(6-aminohexansäure)

### 5.1 Herstellung des Zwischenprodukts N,N'-[6-Chlor-1,3,5-triazin-2,4-diyl]-bis-(6-aminohexansäure)

siehe Beispiel 4.1

### 5.2 Herstellung des Rohprodukts

186,9 g des Zwischenprodukts hergestellt gemäß Beispiel 5.1 (0,5 mol) wurden in einem 2-1-Glasreaktionskolben in 1 1 Wasser mit 65,1 g 3-(Diethylamino)-1-propylamin (0,5 mol) bei 25 bis 40 °C und mit 80,0 g einer 25 Gew.-%igen Natronlauge (entspricht 0,5 mol Natriumhydroxid) bei 35 bis 40 °C umgesetzt und 3 h lang bei 101 bis 103 °C erhitzt. Nach Abkühlen und Entspannen wurden 1332 g Filtrat erhalten.

### 5.3 Aufarbeitung des Rohprodukts

Analog zu Beispiel 1.3 b.) wurde das Filtrat über 1000 ml Amberlyst 35 (1,9 mol H⁺) entsalzt und gereinigt. Die Ausbeute betrug 222,4 g (0,48 mol, 95 % bezogen auf die eingesetzte Menge an Zwischenprodukt 5.1).

**Analyse des Produkts mittels HPLC (CLND):**

| **Zusammensetzung des Produkts** | **[in Gew.-%]** |
|---|---|
| Erfindungsgemäße Triazinverbindung (gemäß Formel (1) mit R₁ gemäß Formel (7c) und R₂, mit E = -NH- und n=5) | 100,0 |
| *R₂ mit m = 0;* *R₃* = *R₂, wobei E* = *-NH-, n=5 und m* = 0 | 91,7 |
| *R₂ mit m = 1-3;* *R₃* = *R₂, wobei E* = *-NH-, n = 5 und m* = *0-3* | *6,9* |
| *R₂ mit m = 0-1;* *R₃ = -OH* | *1,4* |

### Beispiel 6 Herstellung von N-[4,6-Bis-(N-[2,2,6,6-tetramethyl-4-piperidyl-amino])-1,3,5-triazin-2-yl]-6-aminohexansäure

### 6.1 - 6.2 Herstellung des Rohprodukts

In einem 1-1-Glaskolben werden 46,1 g Cyanurchlorid (0,25 mol) in 300 ml Xylol (Isomerengemisch) mit 78,2 g 4-Amino-2,2,6,6-tetramethylpiperidin (0,5 mol) bei 5 bis 20 °C und mit 80,0 g einer 25 Gew.-%iger Natronlauge (entspricht 0,5 mol Natriumhydroxid) bei 20 bis 25 °C umgesetzt und eine Stunde bei 60 °C gerührt. Parallel wurden in einem 250 ml-Glaskolben 56,6 g ε-Caprolactam (0,5 mol) mit 40,0 g einer 25 Gew.-%iger Natronlauge (entspricht 0,25 mol Natriumhydroxid) eine Stunde bei 120 °C umgesetzt und die geschmolzene Lösung aus Aminocapronsäure und ε-Caprolactam wurde in die zweiphasige Suspension zugegeben. Diese Reaktionsmischung wurde unter Rückfluss erhitzt und Wasser über einen Abscheider azeotrop bis zu einer Reaktionstemperatur von 130 °C abdestilliert und weitere 3 Stunden lang bei 127 bis 134 °C unter Atmosphäredruck erhitzt. Nach dem Abkühlen auf 100 °C wurden 250 ml Wasser zugegeben und 1 h unter Rückfluss erhitzt. Bei 25 °C wurde die zweiphasige Suspension filtriert und die Phasen getrennt. Die organische Phase kann für einen Folgeansatz wieder verwendet werden. Es wurden 466 g an wässriger Phase erhalten.

### 6.3 Aufarbeitung des Rohprodukts

Analog zu Beispiel 1.3 b.) wurde das Filtrat über 1040 ml Amberlyst 35 (1,96 mol H⁺) entsalzt und gereinigt. Die Ausbeute betrug 122,7 g (0,23 mol, 95 % bezogen auf die eingesetzte Menge an Cyanurchlorid).

**Analyse des Produkts mittels HPLC (CLND):**

| **Zusammensetzung des Produkts** | **[in Gew.-%]** |
|---|---|
| Erfindungsgemäße Triazinverbindung (gemäß Formel (1) mit R₁ gemäß Formel (7b) und R₂ mit E = -NH- und n = 5) | 100,0 |
| *R₂ mit m = 0, R₃ = R₁* | *92,0* |
| *R₂ mit m* = *1-3, R₃* = *R₁* | *8,0* |

### Beispiel 7 Herstellung von N-[4,6-Bis-(N-[2,2,6,6-tetramethyl-4-piperidyl-amino])-1,3,5-triazin-2-yl]-6-aminohexansäure

### 7.1 - 7.2 Herstellung des Rohprodukts

In einem 2-1-Hastelloy-Autoklav wurden 46,1 g Cyanurchlorid (0,25 mol) in 480 ml Wasser mit 78,2 g 4-Amino-2,2,6,6-tetramethylpiperidin (0,5 mol) bei 5 bis 20 °C und mit 80,0 g einer 25 Gew.-%igen Natronlauge (entspricht 0,5 mol Natriumhydroxid) bei 20 bis 25 °C umgesetzt. Diese Suspension wurde eine Stunde bei 60 °C gerührt. Parallel wurden in einem 250 ml-Glaskolben 56,6 g ε-Caprolactam (0,5 mol) mit 40,0 g einer 25 Gew.-%igen Natronlauge (entspricht 0,25 mol Natriumhydroxid) eine Stunde bei 120 °C umgesetzt und die geschmolzene Lösung aus Aminocapronsäure und ε-Caprolactam in die wässrige Suspension zugegeben. Der Autoklav wurde verschlossen und 4 Stunden bei 153 °C und 6,1 bar erhitzt. Nach Abkühlen und Entspannen wurde die Suspension filtriert. Es wurden 778 g Filtrat erhalten.

### 7.3 Aufarbeitung des Rohprodukts

Analog zu Beispiel 1.3 b.) wurde das Filtrat über 1040 ml Amberlyst 35 (1,96 mol H⁺) entsalzt und gereinigt. Die Ausbeute betrug 112,2 g (0,22 mol, 87 % bezogen auf die eingesetzte Menge an Cyanurchlorid).

**Analyse des Produkts mittels HPLC (CLND):**

| **Zusammensetzung des Produkts** | **[in Gew.-%]** |
|---|---|
| Erfindungsgemäße Triazinverbindung (gemäß Formel (1) mit R₁ gemäß Formel (7b) und R₂ mit E = -NH-, n = 5 und m =0) | 100,0 |
| *R₃* = *R₁* | *94,0* |
| *R₃ = -OH* | *6,0* |

### Beispiel 8 Herstellung von N-[4,6-Bis-(N-[2,2,6,6-tetramethyl-4-piperidyl-amino])-1,3,5-triazin-2-yl]-6-aminohexansäure

### 8.1 - 8.2 Herstellung des Rohprodukts

Analog zu Beispiel 7 wurden 46,1 g Cyanurchlorid (0,25 mol) in 300 ml Wasser mit 78,2 g 4-Amino-2,2,6,6-tetramethylpiperidin (0,5 mol) und 80,0 g einer 25 Gew.-%igen Natronlauge (entspricht 0,5 mol Natriumhydroxid) in einem 1-1-Hastelloy-Autoklav umgesetzt. Nach Zugabe von 56,6 g ε-Caprolactam (0,5 mol) und 40,0 g einer 25 Gew.-%igen Natronlauge (entspricht 0,25 mol Natriumhydroxid) bei 25 °C wurde der Autoklav verschlossen und 1 Stunde bei 115 °C und 1,5 bar und danach 4 Stunden lang bei 149 °C und 5,9 bar erhitzt. Nach Abkühlen und Entspannen wurde die Suspension filtriert. Es wurden 599 g Filtrat erhalten.

### 8.3 Aufarbeitung des Rohprodukts

Analog zu Beispiel 1.3 b.) wurde das Filtrat über 1040 ml Amberlyst 35 (1,96 mol H⁺) entsalzt und gereinigt. Die Ausbeute betrug 118,4 g (0,23 mol, 91 % bezogen auf die eingesetzte Menge an Cyanurchlorid).

**Analyse des Produkts mittels HPLC (CLND):**

| **Zusammensetzung des Produkts** | **[in Gew.-%]** |
|---|---|
| Erfindungsgemäße Triazinverbindung (gemäß Formel (1) mit R₁ gemäß Formel (7b) und R₂ mit E = -NH-, n = 5 und m = 0) | 100,0 |
| *R₃ =R₁* | *98,0* |
| *R₃ = -OH* | *2, 0* |

### Beispiel 9 Herstellung von N-[4,6-Bis-(N-[3-(diethylamino)-1-propyl-aminol)-1,3,5-triazin-2-yl]-6-aminohexansäure

### 9.1 - 9.3 Herstellung und Aufarbeitung des Rohprodukts

Analog zu Beispiel 8 wurden 46,1 g Cyanurchlorid (0,25 mol) in 300 ml Wasser mit 65,1 g 3-(Diethylamino)-1-propylamin (0,5 mol) und 80,0 g einer 25 Gew.-%igen Natronlauge (entspricht 0,5 mol Natriumhydroxid) in einem 1-1-Hastelloy-Autoklav umgesetzt. Nach Zugabe von 56,6 g ε-Caprolactam (0,5 mol) und 40,0 g einer 25 Gew.-%igen Natronlauge (entspricht 0,25 mol Natriumhydroxid) wurde der Autoklav verschlossen und 1 Stunde lang bei 115 °C und 1,5 bar und danach 4 Stunden lang bei 150 °C und 6,0 bar erhitzt. Die Reaktionslösung (588 g) wurde in Analogie zu Beispiel 8 mittels Ionenaustauschchromatographie entsalzt und gereinigt. Die Ausbeute betrug 118,4 g (0,23 mol, 93 % bezogen auf die eingesetzte Menge an Cyanurchlorid).

**Analyse des Produkts mittels HPLC (CLND):**

| **Zusammensetzung des Produkts** | **[in Gew.-%]** |
|---|---|
| Erfindungsgemäße Triazinverbindung (gemäß Formel (1) mit R₁, gemäß Formel (7c) und R₂ mit E = -NH-, m = 0 und n = 5) | 100,0 |
| *R₃ =R₁* | *96,8* |
| *R₃ = -OH* | *3,2* |

### Beispiel 10 Herstellung von N-[4,6-Bis-(N-[2,2,6,6-tetramethyl-4-piperidyl]-N-butylamino)-1,3,5-triazin-2-yl]-6-aminohexansäure

Analog zu Beispiel 8 wurden 46,1 g Cyanurchlorid (0,25 mol) in 300 ml Wasser mit 106,2 g 4-N-Butylamino-2,2,6,6-tetramethylpiperidin (0,5 mol) und 80,0 g einer 25 Gew.-%igen Natronlauge (entspricht 0,5 mol Natriumhydroxid) in einem 1-1-Hastelloy-Autoklav umgesetzt. Nach Zugabe von 56,6 g ε-Caprolactam (0,5 mol) und 40,0 g einer 25 Gew.-%igen Natronlauge (entspricht 0,25 mol Natriumhydroxid) wurde der Autoklav verschlossen und 1 Stunde bei 116 °C und 1,5 bar und danach 4 Stunden bei 152 °C und 6,1 bar erhitzt. Die Reaktionslösung (629 g) wurde analog zu Beispiel 8 mittels Ionenaustauschchromatographie entsalzt und gereinigt. Die Ausbeute betrug 138,2 g (0,22 mol, 88 % bezogen auf die eingesetzte Menge an Cyanurchlorid).

**Analyse des Produkts mittels HPLC (CLND):**

| **Zusammensetzung des Produkts** | **[in Gew.-%]** |
|---|---|
| Erfindungsgemäße Triazinverbindung (gemäß Formel (1) mit R₁, gemäß Formel (7a) und R₂ mit E = -NH-, m = 0 und n = 5) | 100,0 |
| *R₃ =R₁* | *98,0* |
| *R₃* = *-OH* | *2,0* |

### Beispiel 11 Herstellung von N,N'-[6-(N-[2,2,6,6-tetramethyl-4-piperidyl-amino])-1,3,5-triazin-2,4-diyl]-bis-(6-aminohexansäure)

In einem 1-1-Hastelloy-Autoklaven wurden 56,6 g ε-Caprolactam (0,5 mol) und 80,0 g einer 25 Gew.%igen Natronlauge (entspricht 0,5 mol Natriumhydroxid) eine Stunde bei 112 bis 118 °C umgesetzt, mit 300 ml Wasser verdünnt, auf 10 °C abgekühlt, mit 46,1 g Cyanurchlorid (0,25 mol) bei 10 bis 20 °C umgesetzt und eine Stunde bei 20 bis 25 °C gerührt. Nach Zugabe von 39,1 g 4-Amino-2,2,6,6-tetramethylpiperidin (0,25 mol) und 40,0 g einer 25 Gew.-%igen Natronlauge (entspricht 0,25 mol Natriumhydroxid) wurde der Autoklav verschlossen und 6 Stunden bei 150 °C und 6,0 bar erhitzt. Die Reaktionslösung (562 g) wurde analog zu Beispiel 8 mittels Ionenaustauschchromatographie entsalzt und gereinigt. Die Ausbeute betrug 108,8 g (0,22 mol, 86 % bezogen auf die eingesetzte Menge an Cyanurchlorid).

**Analyse des Produkts mittels HPLC (CLND):**

| **Zusammensetzung des Produkts** | **[in Gew.-%]** |
|---|---|
| Erfindungsgemäße Triazinverbindung (gemäß Formel (1) mit R₁ gemäß Formel (7b) und R₂ mit E = -NH- und n = 5) | 100,0 |
| *R₂ mit m = 0;* *R₃ = R₂, wobei E* = *-NH-, m* = *0 und n = 5* | *95,8* |
| *R₂ mit m = 1-3;* *R₃* = *R₂, wobei E* = *-NH, m* = *1-3 und n = 5* | *3,4* |
| *R₂ mit m = 0;* *R₃* = *-OH* | *0,8* |

### Beispiel 12 Herstellung von N,N'-[6-(N-[3-(diethylamino)-1-propyl-amino])-1,3,5-triazin-2,4-diyl]-bis-(6-aminohexansäure)

Analog zu Beispiel 11 wurden 56,6 g ε-Caprolactam (0,5 mol) und 80,0 g einer 25 Gew.-%igen Natronlauge (entspricht 0,5 mol Natriumhydroxid) in einem 1-1-Hastelloy-Autoklaven eine Stunde bei 112 bis 118 °C umgesetzt, mit 300 ml Wasser verdünnt, mit 46,1 g Cyanurchlorid (0,25 mol) bei 10 bis 20 °C umgesetzt und eine Stunde bei 20 bis 25 °C gerührt. Nach Zugabe von 32,6 g 3-(Diethylamino)-1-propylamin (0,25 mol) und 40,0 g einer 25 Gew.%igen Natronlauge (entspricht 0,25 mol Natriumhydroxid) wurde der Autoklav verschlossen und 6 Stunden bei 150 °C und 6,0 bar umgesetzt. Die Reaktionslösung (555 g) wurde analog zu Beispiel 8 mittels Ionenaustauschchromatographie entsalzt und gereinigt. Die Ausbeute betrug 104,0 g (0,22 mol, 88 % bezogen auf die eingesetzte Menge an Cyanurchlorid).

**Analyse des Produkts mittels HPLC (CLND):**

| **Zusammensetzung des Produkts** | **[in Gew.-%]** |
|---|---|
| Erfindungsgemäße Triazinverbindung (gemäß Formel (1) mit R₁ gemäß Formel (7c) und R₂, R₃ mit E = -NH- und n = 5) | 100,0 |
| *R₂ mit m = 0;* *R₃ = R₂, wobei E = -NH-, m = 0 und n = 5* | *96,2* |
| *R₂ mit m = 1-3;* *R₃ = R₂, wobei E = -NH, m = 1-3 und n = 5* | *3,3* |
| *R₂ mit m = 0;* *R₃ = -OH* | *0,5* |

### Beispiel 13 Herstellung von N,N'-[6-(N-[2,2,6,6-tetramethyl-4-piperidyl]-N-butylamino)-1,3,5-triazin-2,4-diyl]-bis-(6-aminohexansäure)

Analog zu Beispiel 11 wurden 56,6 g ε-Caprolactam (0,5 mol) und 80,0 g einer 25 Gew.-%igen Natronlauge (entspricht 0,5 mol Natriumhydroxid) in einem 1-1-Hastelloy-Autoklaven eine Stunde bei 112 bis 118 °C umgesetzt, mit 300 ml Wasser verdünnt, mit 46,1 g Cyanurchlorid (0,25 mol) bei 10 bis 20 °C umgesetzt und eine Stunde bei 20 bis 25 °C gerührt. Nach Zugabe von 53,1 g 4-N-Butylamino-2,2,6,6-tetramethylpiperidin (0,25 mol) und 40,0 g einer 25 Gew.-%igen Natronlauge (entspricht 0,25 mol Natriumhydroxid) wurde der Autoklav verschlossen und 6 Stunden bei 150 °C und 6,0 bar umgesetzt. Die Reaktionslösung (578 g) wurde analog zu Beispiel 8 mittels Ionenaustauschchromatographie entsalzt und gereinigt. Die Ausbeute betrug 124,4 g (0,23 mol, 91 % bezogen auf die eingesetzte Menge an Cyanurchlorid).

**Analyse des Produkts mittels HPLC (CLND):**

| **Zusammensetzung des Produkts** | **[in Gew.-%]** |
|---|---|
| Erfindungsgemäße Triazinverbindung (gemäß Formel (1) mit R₁ gemäß Formel (7a) und R₂ mit E = -NH- und n = 5) | 100,0 |
| *R₂ mit m = 0;* *R₃* = *R₂, wobei E* = *-NH-, m* = *0 und n = 5* | *94,9* |
| *R₂ mit m* = *1-3;* *R₃* = *R₂, wobei E* = *-NH, m* = *1-3 und n = 5* | *4,1* |
| *R₂ mit m = 0;* *R₃ = -OH* | *1,0* |

### Beispiel 14 Herstellung von N-[4,6-Bis-(N-[2,2,6,6-tetramethyl-4-piperidyl-amino])-1,3,5-triazin-2-yl]-6-aminohexansäure

### 14.1 - 14.2 Herstellung des Rohprodukts

Analog zu Beispiel 7 wurden 46,1 g Cyanurchlorid (0,25 mol) in 300 ml Wasser mit 78,2 g 4-Amino-2,2,6,6-tetramethylpiperidin (0,5 mol) und 80,0 g einer 25 Gew.-%igen Natronlauge (entspricht 0,5 mol Natriumhydroxid) in einem 1-1-Hastelloy-Autoklav bei 20 bis 25 °C umgesetzt. Diese Suspension wurde eine Stunde bei 60 °C gerührt. Parallel wurden in einem 250 ml-Glaskolben 54,3 g ε-Laurinlactam (0,28 mol) und 40,0 g einer 25 Gew.-%igen Natronlauge (entspricht 0,25 mol Natriumhydroxid) eine Stunde bei 120 °C umgesetzt und die geschmolzene Lösung aus 1-Aminododecansäure und ε-Laurinlactam in die wässrige Suspension zugegeben. Der Autoklav wurde verschlossen und 4 Stunden bei 153 °C und 6,1 I bar erhitzt. Nach Abkühlen und Entspannen wurde die Suspension filtriert. Es wurden 780 g Filtrat erhalten.

### 14.3 Aufarbeitung des Rohprodukts

Analog zu Beispiel 1.3 b.) wurde das Filtrat über 1040 ml Amberlyst 35 (1,96 mol H⁺) entsalzt und gereinigt. Die Ausbeute betrug 129,3 g (0,21 mol, 86 % bezogen auf die eingesetzte Menge an Cyanurchlorid).

**Analyse des Produkts mittels HPLC (CLND):**

| **Zusammensetzung des Produkts** | **[in Gew.-%]** |
|---|---|
| Erfindungsgemäße Triazinverbindung (gemäß Formel (1) mit R₁ gemäß Formel (7b) und R₂ mit E = -NH- und n = 11 und m =0) | 100,0 |
| *R₃ = R₁* | *95,2* |
| *R₃ = -OH* | *4,8* |

## Patentansprüche

1. Triazinverbindungen der Formel 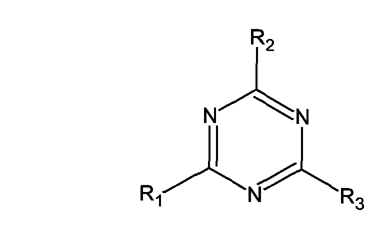 wobei
R₁ = -A-B mit A = -0- oder -NR₄-,
B = Aminogruppe-haltiger Substituent und
R₄ = Wasserstoff oder Alkylgruppe
R₂ = 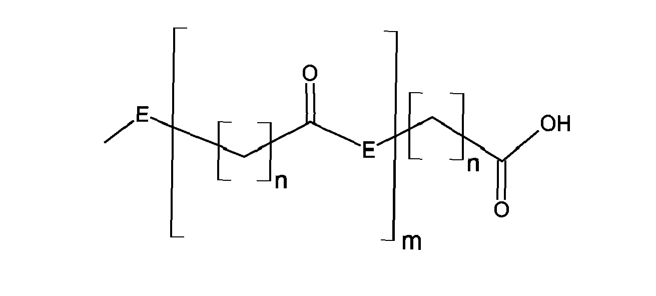 mit E = -O- oder -NR₅-,
n = 3 bis 15,
m = 0 bis 10 und
R₅ = Wasserstoff oder Alkylgruppe
R₃ = R₁, R₂, -OR₆ oder -NR₇R₈
mit R₆, R₇ und R₈ = Wasserstoff, Alkyl- oder Arylgruppe, die jeweils substituiert oder unsubstituiert ist,
ist.

2. Triazinverbindungen gemäß Anspruch 1,
**dadurch gekennzeichnet,**
**dass** die Triazinverbindungen einen Substituenten vom Typ R₁ mit einem Substituenten vom Typ B gemäß der Formel 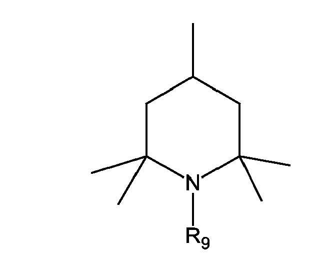 mit R₉ = Wasserstoff, Alkyl- oder eine Alkoxygruppe der Formel -O-R₂₀,
R₂₀ = verzweigte oder unverzweigte Alkyl- oder Cycloalkylgruppe mit jeweils einer Kohlenstoffanzahl von 4 bis 16, ist,
oder gemäß der Formel
-(CH₂)ₚ-NR₁₀R₁₁
mit p von 1 bis 15 und
R₁₀, R₁₁ = Wasserstoff, Alkyl-, Cycloalkyl- oder
Heterocycloalkylgruppe
und R₁₀ und R₁₁ gleich oder verschieden ist,
aufweisen.

3. Triazinverbindungen gemäß Anspruch 2,
**dadurch gekennzeichnet,**
**dass** die Triazinverbindungen einen Substituenten vom Typ R₁ gemäß der Formel 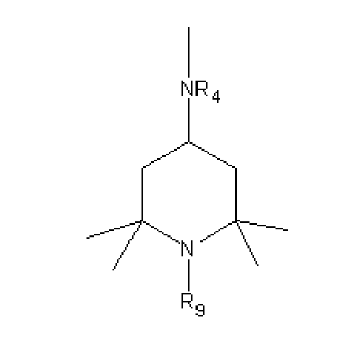 mit R₉ = Wasserstoff, Alkyl- oder eine Alkoxygruppe der Formel -O-R₂₀,
R₂₀ = verzweigte oder unverzweigte Alkyl- oder Cycloalkylgruppe mit jeweils einer Kohlenstoffanzahl von 4 bis 16, ist,
R₄ = Wasserstoff oder Alkylgruppe,
aufweisen.

4. Triazinverbindungen gemäß zumindest einem der Ansprüche 1 bis 3,
**dadurch gekennzeichnet,**
**dass** die Triazinverbindungen als Substituenten vom Typ R₃ einen Substituenten vom Typ R₁ aufweisen.

5. Triazinverbindungen gemäß zumindest einem der Ansprüche 1 bis 3,
**dadurch gekennzeichnet,**
**dass** die Triazinverbindungen als Substituenten vom Typ R₃ einen Substituenten vom Typ R₂ aufweisen.

6. Verfahren zur Herstellung von Triazinverbindungen gemäß der Formel, 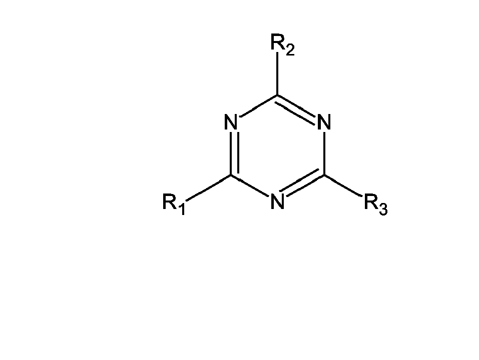 wobei
R₁ = -A-B mit A = -O- oder -NR₄-,
B = Aminogruppe-haltiger Substituent und
R₄ = Wasserstoff oder Alkylgruppe
R₂ = 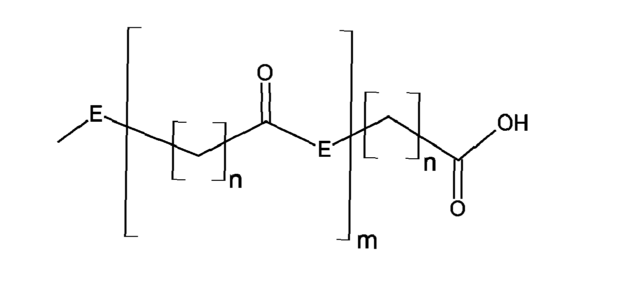 mit E = -O- oder -NR₅-,
n = 3 bis 15,
m = 0 bis 10 und
R₅ = Wasserstoff oder Alkylgruppe
R₃ = R₁, R₂, -OR₆ oder -NR₇R₈
mit R₆, R₇ und R₈ = Wasserstoff, Alkyl- oder Arylgruppe, die jeweils substituiert oder unsubstituiert ist,
ist,
**dadurch gekennzeichnet,**
**dass** Cyanurchlorid mit von 0,5 bis 5 Moläquivalenten eines Amins der Formel
H-A-B
in Gegenwart einer Base und von 0,5 bis 5 Moläquivalenten einer Verbindung gemäß der Formel 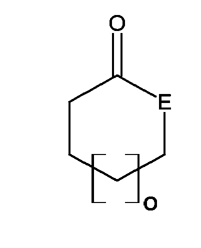 oder einer Verbindung gemäß der Formel 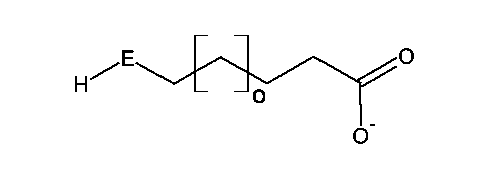 mit o von 0 bis 12,
E = -0- oder -NR₅- und
R₅ = Wasserstoff oder Alkylgruppe,
umgesetzt wird, wobei die Reihenfolge der beiden Reaktionsschritte beliebig ist.

7. Verfahren gemäß Anspruch 6,
**dadurch gekennzeichnet,**
**dass** in einem weiteren Reaktionsschritt 0,5 bis 5 Moläquivalente einer Verbindung gemäß den Formeln
H-OR₆ oder H-NR₇R₈
in Gegenwart einer Base umgesetzt werden.

8. Zusammensetzung,
**dadurch gekennzeichnet,**
**dass** die Zusammensetzung zumindest zwei verschiedene Triazinverbindungen gemäß der Formel 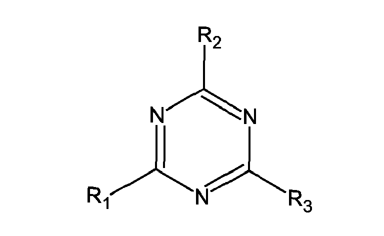 wobei
R₁ = -A-B mit A = -0- oder -NR₄-,
B = Aminogruppe-haltiger Substituent und
R₄ = Wasserstoff oder Alkylgruppe
R₂ = 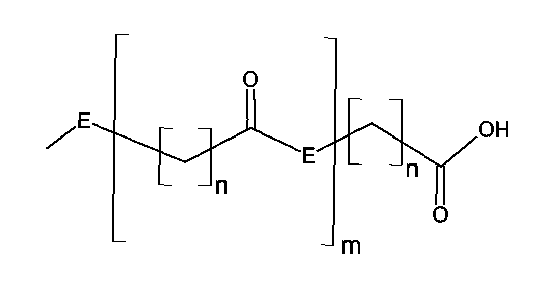 mit E = -O- oder -NR₅-,
n = 3 bis 15,
m = 0 bis 10 und
R₅ = Wasserstoff oder Alkylgruppe
R₃ = R₁, R₂, -OR₆ oder -NR₇R₈
mit R₆, R₇ und R₈ = Wasserstoff, Alkyl- oder Arylgruppe, die jeweils substituiert oder unsubstituiert ist,
ist,
aufweist.

9. Lösung,
**dadurch gekennzeichnet,**
**dass** die Lösung zumindest eine Triazinverbindung gemäß der Formel 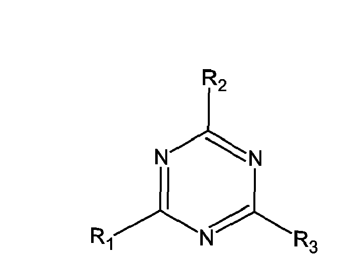 wobei
R₁ = -A-B mit A = -O- oder -NR₄-,
B = Aminogruppe-haltiger Substituent und
R₄ = Wasserstoff oder Alkylgruppe
R_{2 =} 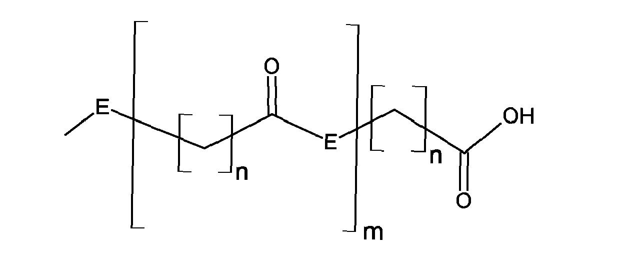 mit E = -0- oder -NR₅-,
n = 3 bis 15,
m = 0 bis 10 und
R₅ = Wasserstoff oder Alkylgruppe
R₃ = R₁, R₂, -OR₆ oder -NR₇R₈
mit R₆, R₇ und R₈ = Wasserstoff, Alkyl- oder Arylgruppe, die jeweils substituiert oder unsubstituiert ist,
ist,
aufweist.

10. Lösung gemäß Anspruch 9,
**dadurch gekennzeichnet,**
**dass** die Lösung als Lösemittel Wasser aufweist.

11. Lösung gemäß Anspruch 9 oder 10,
**dadurch gekennzeichnet,**
**dass** die Lösung von 1 bis 50 Gew.-% an Triazinverbindungen aufweist.
